# EUROPEAN PATENT APPLICATION

(11) **EP 4 299 078 A1**
(43) Date of publication of application: **03.01.2024**
(21) Application number: 22760104.4
(22) Date of filing: 25.02.2022
(51) Int. Cl.: A61K 48/00, A61K 31/7105, A61K 31/713, A61K 9/51, A61K 47/60, A61K 47/58, A61K 47/54, A61P 17/14, A61K 8/60

(54) **COMPOSITION FOR ALLEVIATING HAIR GRAYING, PROMOTING HAIR GROWTH AND/OR PREVENTING OR ALLEVIATING HAIR LOSS, COMPRISING DOUBLE-STRANDED MIRNA AS ACTIVE INGREDIENT**

(30) Priority: 25.02.2021 KR 20210025554
(71) Applicant: Bioneer Corporation, Daejeon 34302 (KR); Sirnagen Therapeutics Corporation, Daejeon 34302 (KR)
(72) Inventor: PARK, Han-Oh, Sejong 30151 (KR); LEE, Sang-Kyu, Daejeon 34016 (KR); KWON, Oh Seung, Sejong 30126 (KR); GOH, Eun-Ah, Sejong 30150 (KR)
(74) Representative: Mewburn Ellis LLP
(86) International application number: PCT/KR2022/002765
(87) International publication number: WO 2022/182188

(57) **Abstract**

The present invention relates to a composition for alleviating hair graying, promoting hair growth and/or preventing or alleviating hair loss, comprising double-stranded miRNA as an active ingredient and, more specifically, to: miR-3139, miR-3189, miR-3199 or miR-8485, which are double-stranded miRNA; a double-stranded oligonucleotide structure comprising same; or a pharmaceutical or cosmetic composition for alleviating hair graying, promoting hair growth and/or preventing or alleviating hair loss, comprising nanoparticles containing the structure as an active ingredient. A composition according to the present invention can prevent hair graying and reduce the rate of progress thereof by activating melanocytes and promoting melanogenesis and can allow the hair already affected by graying to be improved to a state before graying. In addition, the composition promotes the activation of melanocytes and the proliferation of dermal papilla cells and keratinocytes that are present in hair follicles, and induces outer root sheath development and hair growth so that the effects of promoting hair growth and/or preventing hair loss can be exhibited.

## Description

### [Technical Field]

The present invention relates to a composition for alleviating hair graying, promoting hair growth, and/or preventing or alleviating hair loss, containing double-stranded miRNA as an active ingredient, and more particularly to a pharmaceutical or cosmetic composition for alleviating hair graying, promoting hair growth, and/or preventing or alleviating hair loss, containing, as an active ingredient, double-stranded miRNA such as miR-3139, miR-3189, miR-3199, or miR-8485, a double-stranded oligonucleotide construct including the same, or nanoparticles including the construct.

### [Background Art]

Hair graying in humans is a phenomenon of physiological aging, and is known to be caused by a decrease in melanin pigment due to functional deterioration and depletion of melanocytes or melanocyte stem cells in hair follicles. Melanin pigment is an important factor that determines the color of hair, and melanin is synthesized in and secreted from the melanosomes present in melanocytes, transferred to nearby keratinocytes, fibroblasts, etc., and then moves along with hair growth and maintains the color of the hair. Currently, a simple dyeing procedure is used as a solution to hair graying, but dyeing is a temporary method and re-dyeing is essential due to growth of white hair, and ingredients contained in hair dyes have various side effects, such as irritation to the scalp and hair follicles, or causing allergies or dermatitis. Current research related to melanin pigment production control is limited to development of materials exhibiting skin whitening effects and identification of mechanisms of action thereof by suppressing symptoms such as pigmentation, etc. due to overproduction of melanin in the skin caused by various factors such as genetic factors, aging, hormones, growth factors, ultraviolet light, and the like. Conversely, little research has been conducted on the mechanism of action and development of materials related to promotion of melanogenesis to overcome hair graying or decreased melanin synthesis. Moreover, melanogenesis is regulated by interactions of various signals (SCF-KIT-RAS-ERK signaling pathway, MC1R-cAMP-PKA signaling pathway, Wnt-β-catenin signaling pathway). Due to such a complicated mechanism of action, it is not easy to identify the mechanism of action and develop new materials in order to prevent or alleviate graying of hair.

Recently, androgenetic alopecia (AGA), which mainly occurs in middle-aged men and is considered a genetic disease of men, is common in women as well as young men due to severe environmental pollution, stress, dietary changes, and rapid aging of the population. As interest in hair loss increases, the hair loss market is growing rapidly. It is known that hair loss is caused by various factors such as genetic factors, male hormones, aging, blood circulation disorders, and the like. Currently approved drugs only include finasteride, which inhibits the production of dihydrotestosterone (DHT) as a male hormone (androgen), and minoxidil, which improves blood vessel dilation and blood circulation. However, these drugs have temporary and limited efficacy depending on the dose and administration mode, and are accompanied by various side effects. Hence, development of new materials that induce the activation of hair follicle cells and cell proliferation to promote hair growth without side effects is required.

The hair cycle includes three stages: an anagen phase in which hair grows most actively, a catagen phase in which hair growth stops and hair degeneration begins, and a telogen phase in which hair follicle activity is suspended, and hair loss refers to natural loss of hair that has stopped growing according to the growth cycle, and it is known that various factors cause hair loss. Although drug development for alleviating symptoms of hair loss has been made for a long time, there are only finasteride, which is an oral hair loss therapeutic agent, and minoxidil, which is a topical hair loss therapeutic agent. However, since these drugs induce the relief of hair loss symptoms through the mechanism of inhibiting production of male hormones, they have side effects and limitations in efficacy. Therefore, it is necessary to develop materials with new mechanisms and strategies that exhibit efficacy without side effects. It is an important strategy for development of hair loss therapeutic agents to induce rapid conversion of hair from the telogen phase to the anagen phase by promoting the activation and proliferation of hair follicle cells. Accordingly, it is required to develop new materials that induce activation of hair follicle cells and cell proliferation to promote hair growth.

Technology for suppressing gene expression is an important tool in development of therapeutic agents and target verification for disease treatment, and development has been carried out in various ways to overcome limitations of conventional drug treatment methods, one of which is use of RNA interference (hereinafter referred to as "RNAi") (Iorns, E., Lord, C.J., Turner, N. & Ashworth, A., Nat. Rev. Drug Discov. 6, 556-68. 2007). RNAi is a method used to suppress gene expression and the field of application thereof is diversifying because it is simple and clearly shows lowcost gene suppression effects. siRNA is single-stranded RNA composed of 16 to 27 nucleotides and acts as a component of a ribonucleoprotein known as RISC (RNA induced silencing complex) in cells. RISC functions as RNA enzyme scissors and cleaves messenger RNA (hereinafter referred to as "mRNA") to inhibit protein production from mRNA. siRNA included in RISC binds to mRNA having a sequence complementary to the siRNA sequence to form double-stranded RNA, and RISC serving as RNA enzyme scissors cleaves target mRNA so that mRNA is no longer able to function as a template for protein production.

As such, RNAi-based therapeutics are evaluated as more advanced than single-molecule therapeutics in that they block mRNA before the protein production stage and utilize RNA and the cell-intrinsic RISC system, but cause side effects that cannot be resolved even with siRNA-based technology, which is a phenomenon called the off-target effect. As described above, RNAi-based therapeutics degrade mRNA that binds complementarily to the siRNA sequence, but bind to mRNA that is complementary only to a portion of the siRNA sequence, rather than the entire siRNA sequence, causing degradation, which is referred to as "off-target effect" because degradation of non-target mRNA is induced.

With the goal of overcoming the technical difficulties of the RNAi-based therapeutics described above, thorough research into use of microRNA (hereinafter referred to as "miRNA") as a therapeutic agent is ongoing (Agostini, M. & Knight, R.A., Oncotarget 5, 872-81, 2014; Dangwal, S. & Thum, T., Annu. Rev. PharmacolToxicol. 54, 185-203, 2014). miRNA is RNA composed of 16 to 27 nucleotides and is classified as protein non-coding RNA in preparation for mRNA that is translated into protein (Carthew, R.W. & Sontheimer, E.J., Cell 136, 642-55, 2009; MacFarlane, L.-A. & Murphy, P.R., Current Genomics 11, 537-561, 2010; Bartel, D.P., Cell 136, 215-33, 2009). miRNA is recorded in genomes of higher animal and plant cells and is known to play a key role in regulating cell metabolism and functions including cell production, growth, differentiation, and death. About 2000 types of miRNAs are known to date in the human genome, and the functions of considerable numbers of miRNAs there among are still unknown.

miRNA is transcribed into RNA by an RNA polymerase called Pol II from the genome, and the initial length thereof is not specified and varies (Carthew, R.W. & Sontheimer, E.J., Cell 136, 642-55, 2009; Brodersen, P. & Voinnet, O., Nat. Rev. Mol. Cell Biol. 10, 141-148, 2009). This is due to the diversity of positions where miRNA is included in the genome, particularly due to production of miRNA in various ways, such as being located in an intron, which is a part of mRNA that is not involved in protein production and transcribed at the same time as mRNA is produced, or being located in an intergenic region on the genome and independently transcribed (Malone, C.D. & Hannon, G.J., Cell 136, 656-68, 2009). The miRNA matrix initially generated in this way is called primary miR (primary microRNA), and primary miRNA is edited into precursor miRNA (hereinafter referred to as "pre-miR") by RNase called Drosha in the nucleus (Bartel, D.P., Cell 136, 215-33, 2009). Pre-miR forms an RNA hair-pin structure and is composed of approximately 70-80 nucleotides. Pre-miR inside the cell nucleus is moved from the nucleus to the cytoplasm by exportin proteins, etc., and is secondarily processed by another RNase called Dicer in the cytoplasm to form double-stranded mature miRNA composed of 16-27 nucleotides (mature microRNA, hereinafter, miR described without other modifiers indicates mature miR). In double-stranded miR, one-strand RNA is selected, binds to RISC that is a ribonucleoprotein and thus becomes active, and binds to the target mRNA using the sequence of miR.

In general, mRNA may be largely divided into three regions based on whether they are involved in protein production: a coding region that contains protein translation information, and 5'-UTR (un-translated region) and 3'-UTR that do not have protein translation information as respective 5' and 3' ends of the coding region. siRNA that induces degradation of target mRNA using complementary sequences to mRNA acts regardless of the 5' and 3'-UTRs and coding region of mRNA, whereas miR mainly binds to the 3'-UTR (Carthew, R.W. & Sontheimer, E.J., Cell 136, 642-55, 2009; Bartel, D.P., Cell 136, 215-33, 2009). A unique feature of siRNA and miRNA, in addition to the difference in binding position to mRNA, is that siRNA binds mainly to mRNA including the sequence complementary to the entire sequence of siRNA, whereas miRNA is configured such that a limitedsize seed region sequence located 2-8 nucleotides from the 5' end of miRNA is mainly used for target mRNA recognition, and thus, even when the entire miRNA sequence does not have a perfectly complementary sequence to the target gene and includes a certain portion of non-complementary sequence, miRNA activity is not affected (Bartel, D.P., Cell 136, 215-33, 2009). As the seed region sequence is 6-8 nucleotides long, there are various types of mRNAs having sequences complementary thereto in the 3'-UTR, and hence, a single type of miRNA is able to simultaneously control several types of mRNAs. Such miRNA functions as an efficient regulator involved in the control of many aspects of cell physiology including cell division, growth, differentiation, and death. Also, the function of miRNA as the regulator has the advantage of being able to be effectively applied to various diseases. In contrast to siRNA, which aims to suppress the expression of a single gene, miRNA is able to simultaneously inhibit the expression of multiple genes involved in various signaling pathways. Large numbers of mRNAs contain a region in the 3'-UTR where at least one miRNA is likely to bind, and based on a bioinformatic calculation, protein production from about 30% of all mRNAs is known to be regulated by miRNA.

Despite excellent effects and wide ranges of use of miRNA or siRNA, in order for miRNA/siRNA to be developed as a therapeutic agent, miRNA/siRNA must be effectively delivered to target cells by improving the stability of miRNA/siRNA in the body and increasing the efficiency of cell delivery (FY Xie., Drug Discov. Today. 2006 Jan; 11(1-2) :67-73) . In order to improve stability in the body and solve the problem of non-specific innate immune stimulation of miRNA/siRNA, thorough research is ongoing into modification of some nucleotides or backbones of miRNA/siRNA to impart nuclease resistance thereto or into use of carriers such as viral vectors, liposomes, nanoparticles, etc. A delivery system using a viral vector such as an adenovirus or a retrovirus has high transfection efficacy, but high immunogenicity and oncogenicity. On the other hand, a nonviral delivery system including nanoparticles has low cell delivery efficiency compared to a viral delivery system, but it is advantageous because of high stability *in vivo,* the potential for target-specific delivery, uptake and internalization of the contained RNAi oligonucleotides into cells or tissues, and almost no cytotoxicity or innate immune stimulation, such that it is currently considered a more powerful delivery method than the viral delivery system (Akhtar S, J. Clin. Invest. 2007 December 3; 117(12): 3623-3632) .

As for the method of using a nanocarrier in the nonviral delivery system, nanoparticles are formed using various polymers such as liposomes, cationic polymer complexes, and the like, and miRNA/siRNA is loaded on such a nanoparticle, namely a nanocarrier, and is delivered to cells. Among the methods of using a nanocarrier, a polymeric nanoparticle, a polymer micelle, a lipoplex, etc. may be mainly used, and in particular, the lipoplex is composed of cationic lipids and interacts with the anionic lipids of the endosomes of cells, causing the effect of destabilization of the endosomes to thus enable intracellular delivery. Moreover, high efficiency *in vivo* is known to be inducible by linking a chemical material to the end of the miRNA/siRNA sense (passenger) strand to exhibit enhanced pharmacokinetics (J. Soutschek, Nature 11; 432(7014) :173-8, 2004). As such, stability of miRNA/siRNA varies depending on the properties of the chemical material bound to the end of the miRNA/siRNA sense or antisense (guide) strand.

For example, siRNA with a polymer compound such as polyethylene glycol (PEG) conjugated interacts with the anionic phosphate group of miRNA/siRNA in the presence of a cationic material to form a complex, resulting in a carrier having improved miRNA/siRNA stability (S.H. Kim, J. Control Release 129(2):107-16, 2008). In particular, micelles composed of polymer complexes are extremely small in size compared to other systems used as drug delivery vehicles, such as microspheres or nanoparticles, but have a very regular distribution and are spontaneously formed, so that easy quality control and reproducibility of formulations may be realized. In order to improve the intracellular delivery efficiency of miRNA/siRNA, technology for attaining the stability of miRNA/siRNA and efficient cell membrane permeability has been developed using a miRNA/siRNA conjugate in which a hydrophilic material (e.g. polyethylene glycol (PEG)) as a biocompatible polymer is conjugated to miRNA/siRNA through a simple covalent bond or a linker-mediated covalent bond (Korean Patent No. 883471).

However, chemical modification of miRNA/siRNA and conjugation of polyethylene glycol (PEG) (PEGylation) still have drawbacks such as low stability *in vivo* and inefficient delivery to target organs. In order to solve these drawbacks, a double-stranded oligonucleotide construct, in which hydrophilic and hydrophobic materials bind to a double-stranded oligonucleotide, particularly double-stranded oligo RNA such as miRNA/siRNA, has been developed, and the construct forms self-assembled nanoparticles called SAMiRNA^{™} (self-assembled micelle inhibitory RNA) through hydrophobic interaction of hydrophobic materials (Korean Patent No. 1224828). SAMiRNA^{™} technology has the advantage of being able to obtain homogenous nanoparticles while being very small in size compared to conventional delivery techniques.

As for a specific example of SAMiRNA^{™} technology, PEG (polyethylene glycol) or HEG (hexaethylene glycol) is used as a hydrophilic material, and PEG is a synthetic polymer and is often used to increase the solubility of pharmaceuticals, particularly proteins, and to control pharmacokinetics. PEG is a polydisperse material, and a batch of polymers is composed of the total sum of different numbers of monomers, and has a Gaussian molecular weight distribution, and the extent of homogeneity of a material is represented as a polydispersity index (Mw/Mn). Specifically, when PEG has a low molecular weight (3-5 kDa), it exhibits a polydispersity index of about 1.01, whereas the case of a high molecular weight (20 kDa) shows a high polydispersity index of about 1.2, and thus the higher the molecular weight, the lower the homogeneity of the material. Therefore, the case in which PEG binds to a pharmaceutical is disadvantageous in that it is not easy to verify a single material because the characteristic polydispersity of PEG is reflected in the conjugate. Hence, there is a trend to produce materials having a low polydispersity index by improving the processes for synthesis and purification of PEG. In particular, in the case in which PEG binds to a material having a low molecular weight, there are problems due to the polydispersity of the material, such as an inconvenient point in which it is not easy to check whether binding is easily achieved (Francesco M. VDRUG DISCOVERY TODAY(2005) 10(21) :1451-1458). Thus, in recent years, as an improved form of existing self-assembled nanoparticles SAMiRNA^{™}, the hydrophilic material of the double-stranded oligonucleotide construct constituting SAMiRNA^{™} is blocked into a basic unit including 1 to 15 homogeneous monomers having a certain molecular weight, and, as necessary, a linker, and by using an appropriate number of blocks depending on the need, a new form of delivery carrier technology has been developed that has remarkably improved polydispersity with a small size as compared to existing SAMiRNA^{™}.

Accordingly, the present inventors have made great efforts to discover miRNA capable of alleviating hair graying, and ascertained that miR-3139, miR-3189, miR-3199, or miR-8485 may activate melanocytes and promote melanogenesis, thus alleviating graying of hair and also that proliferation of hair follicle dermal papilla cells, keratinocytes, etc. is stimulated in addition to melanocyte activation, and the outer root sheath (ORS) of hair follicles and hair length are increased, thus culminating in the present invention.

### Patent Literature

(Patent Document 1) Korean Patent No. 883471
(Patent Document 2) Korean Patent No. 1224828
(Patent Document 3) Korean Patent No. 1862349

### Non-Patent Literature

(Non-Patent Document 1) Iorns, E., Lord, C.J., Turner, N. & Ashworth, A., Nat. Rev. Drug Discov. 6, 556-68. 2007
(Non-Patent Document 2) Agostini, M. & Knight, R.A., Oncotarget 5, 872-81, 2014
(Non-Patent Document 3) Dangwal, S. & Thum, T., Annu. Rev. PharmacolToxicol. 54, 185-203, 2014
(Non-Patent Document 4) Carthew, R.W. & Sontheimer, E.J., Cell 136, 642-55, 2009
(Non-Patent Document 5) MacFarlane, L.-A. & Murphy, P.R., Current Genomics 11, 537-561, 2010
(Non-Patent Document 6) Bartel, D.P., Cell 136, 215-33, 2009
(Non-Patent Document 7) Carthew, R.W. & Sontheimer, E.J., Cell 136, 642-55, 2009
(Non-Patent Document 8) Brodersen, P. & Voinnet, O., Nat. Rev. Mol. Cell Biol. 10, 141-148, 2009
(Non-Patent Document 9) Malone, C.D. & Hannon, G.J., Cell 136, 656-68, 2009
(Non-Patent Document 10) Akhtar S, J. Clin Invest. 2007 December 3; 117(12): 3623-3632, 2007
(Non-Patent Document 11) S.H. Kim, J. Control Release 129 (2) :107-16, 2008

### [Disclosure]

It is an object of the present invention to provide a novel composition capable of alleviating hair graying by activating melanocytes of hair follicles and promoting melanogenesis, and preventing or alleviating hair loss and promoting hair growth by inducing proliferation of hair follicle cells.

In order to accomplish the above object, the present invention provides a pharmaceutical composition for alleviating hair graying, promoting hair growth, and/or preventing or alleviating hair loss, including, as an active ingredient, (i) miR-3139, miR-3189, miR-3199, or miR-8485;
(ii) a double-stranded oligonucleotide construct having the structure of Structural Formula (1) below:

   A-X-R-Y-B Structural Formula (1)
in Structural Formula (1), A is a hydrophilic material, B is a hydrophobic material, X and Y each independently represent a simple covalent bond or a linker-mediated covalent bond, and R represents miR-3139, miR-3189, miR-3199, or miR-8485; or
(iii) nanoparticles including the double-stranded oligonucleotide construct.

In addition, the present invention provides a cosmetic composition for alleviating hair graying, promoting hair growth, and/or preventing or alleviating hair loss, including, as an active ingredient, (i) miR-3139, miR-3189, miR-3199, or miR-8485; (ii) a double-stranded oligonucleotide construct having the structure of Structural Formula (1) below:

A-X-R-Y-B Structural Formula (1)

in Structural Formula (1), A is a hydrophilic material, B is a hydrophobic material, X and Y each independently represent a simple covalent bond or a linker-mediated covalent bond, and R represents miR-3139, miR-3189, miR-3199, or miR-8485; or
(iii) nanoparticles including the double-stranded oligonucleotide construct.

In addition, the present invention provides a method of alleviating hair graying, promoting hair growth, and/or preventing or alleviating hair loss including administering, to a subject in need of alleviating hair graying, promoting hair growth, and/or preventing or alleviating hair loss,
(i) miR-3139, miR-3189, miR-3199, or miR-8485;
(ii) a double-stranded oligonucleotide construct having the structure of Structural Formula (1) below:

   A-X-R-Y-B Structural Formula (1)

   in Structural Formula (1), A is a hydrophilic material, B is a hydrophobic material, X and Y each independently represent a simple covalent bond or a linker-mediated covalent bond, and R represents miR-3139, miR-3189, miR-3199, or miR-8485; or
(iii) nanoparticles including the double-stranded oligonucleotide construct.

In addition, the present invention provides the use of, for alleviating hair graying, promoting hair growth, and/or preventing or alleviating hair loss,
(i) miR-3139, miR-3189, miR-3199, or miR-8485;
(ii) a double-stranded oligonucleotide construct having the structure of Structural Formula (1):

   A-X-R-Y-B Structural Formula (1)

   in Structural Formula (1), A is a hydrophilic material, B is a hydrophobic material, X and Y each independently represent a simple covalent bond or a linker-mediated covalent bond, and R represents miR-3139, miR-3189, miR-3199, or miR-8485; or (iii) nanoparticles including the double-stranded oligonucleotide construct.

In addition, the present invention provides the use of, for the manufacture of medicaments or cosmetics for alleviating hair graying, promoting hair growth, and/or preventing or alleviating hair loss,
(i) miR-3139, miR-3189, miR-3199, or miR-8485;
(ii) a double-stranded oligonucleotide construct having the structure of Structural Formula (1) below:

   A-X-R-Y-B Structural Formula (1)

   in Structural Formula (1), A is a hydrophilic material, B is a hydrophobic material, X and Y each independently represent a simple covalent bond or a linker-mediated covalent bond, and R represents miR-3139, miR-3189, miR-3199, or miR-8485; or (iii) nanoparticles including the double-stranded oligonucleotide construct.

### [Description of Drawings]

FIG. 1 shows results of primary screening of 1728 human miRNAs loaded on SAMiRNA^{™} by measurement of the amount of melanin produced in a human skin cancer cell line (M21);
FIG. 2 shows results of secondary screening of 18 miRNAs with a high amount of melanin produced selected through screening in a human skin cancer cell line (M21);
FIG. 3 shows results of evaluating reproducibility for the amount of melanin produced and color change efficacy in a human skin cancer cell line (SK-MEL-28) by 9 miRNAs with the highest amount of melanin produced selected through primary and secondary screening;
FIG. 4 shows results of analyzing changes in melanogenesis signaling through RT-qPCR to analyze the mechanism of action of 9 miRNAs including miR-8485, miR-7978, miR-6074, miR-3132, miR-4644, miR-3139, miR-3189, miR-3199, and miR-933 with the highest amount of melanin produced in an SK-MEL-28 cell line;
FIG. 5 shows results of analyzing changes in melanogenesis signaling through RT-qPCR by 9 selected miRNAs including miR-8485, miR-7978, miR-6074, miR-3132, miR-4644, miR-3139, miR-3189, miR-3199, and miR-933 in a human epidermal melanocyte cell line;
FIG. 6 shows results confirming changes in melanogenesis signaling through Western blotting to analyze the mechanism of action of 9 selected miRNAs including miR-8485, miR-7978, miR-6074, miR-3132, miR-4644, miR-3139, miR-3189, miR-3199, and miR-933 in an SK-MEL-28 cell line;
FIG. 7 shows results of observing outer root sheath (ORS) and hair follicle cell proliferation promotion in plucked human gray hair by the finally selected 4 miRNAs including miR-3139, miR-3189, miR-3199, and miR-8485;
FIG. 8 shows results of observing efficacy (color change) in plucked human gray hair by the finally selected 4 miRNAs including miR-3139, miR-3189, miR-3199, and miR-8485;
FIG. 9a shows results of analyzing cell color change and the amount of melanin produced by treatment of an SK-MEL-28 cell line with SAMiRNA-miR-3199;
FIG. 9b shows results confirming changes in melanogenesis signaling through RT-qPCR by treatment of an SK-MEL-28 cell line with SAMiRNA-miR-3199;
FIG. 9c shows results confirming changes in melanogenesis signaling through immunoblotting by treatment of an SK-MEL-28 cell line with SAMiRNA-miR-3199;
FIG. 9d shows results confirming efficacy of changes in melanogenesis signaling through immunocytochemistry by treatment of an SK-MEL-28 cell line with SAMiRNA-miR-3199;
FIG. 10a shows results of selecting GSK3β as the target gene of miR-3199;
FIG. 10b results confirming the GSK3β inhibitory efficacy through RT-qPCR by treatment of an SK-MEL-28 cell line with SAMiRNA-miR-3199;
FIG. 10c shows results confirming the GSK3β inhibitory efficacy through immunoblotting by treatment of an SK-MEL-28 cell line with SAMiRNA-miR-3199;
FIG. 10d shows results confirming the GSK3β inhibitory efficacy through immunocytochemistry by treatment of an SK-MEL-28 cell line with SAMiRNA-miR-3199;
FIG. 10e shows results confirming the action of miR-3199 by directly binding to GSK3β mRNA 3'-UTR through luciferase reporter assay;
FIG. 11a shows results of analyzing cell color change and the amount of melanin produced by treatment of human epidermal melanocytes with SAMiRNA-miR-3199;
FIG. 11b shows results confirming changes in melanogenesis signaling through RT-qPCR by treatment of human epidermal melanocytes with SAMiRNA-miR-3199;
FIG. 11c shows results confirming changes in melanogenesis signaling through immunoblotting by treatment of human epidermal melanocytes with SAMiRNA-miR-3199;
FIG. 12a shows results of evaluating cytotoxicity of SAMiRNA-miR-3199 in human follicle dermal papilla cells, keratinocytes, and human melanocytes; and
FIG. 12b shows results of evaluating innate immunotoxicity of SAMiRNA-miR-3199 by confirming an increase in inflammatory cytokines in human peripheral blood mononuclear cell (PBMC).

### [Mode for Invention]

Unless otherwise defined, all technical and scientific terms used herein have the same meanings as typically understood by those skilled in the art to which the present invention belongs. In general, the nomenclature used herein is well known in the art and is typical.

In the present invention, due to the demand to prevent or alleviate hair graying and hair loss, a screening library for 1728 human miRNAs was synthesized (Table 2) and used to treat human skin cancer cell lines and human epidermal melanocytes, and the amount of melanin produced was measured and cell color change was analyzed, whereby four effective miRNAs, including miR-3139, miR-3189, miR-3199, and miR-8485, were discovered. In addition, miRNA was confirmed to be able to alleviate hair graying by activating melanocytes and promoting melanogenesis in plucked human gray hair, and the mechanism of action of melanogenesis promotion thereof was identified, and also, through evaluation of cytotoxicity and innate immunotoxicity, miRNA was confirmed to be able to promote melanogenesis in hair without side effects. In addition, it was confirmed that proliferation of cells such as hair follicle dermal papilla cells and keratinocytes was promoted along with melanocyte activation, and outer root sheath was developed and the hair length was increased.

Accordingly, an aspect of the present invention pertains to a pharmaceutical composition for alleviating hair graying, promoting hair growth, and/or preventing or alleviating hair loss, including, as an active ingredient:
(i) miR-3139, miR-3189, miR-3199, or miR-8485;
(ii) a double-stranded oligonucleotide construct having the structure of Structural Formula (1) below:

   A-X-R-Y-B Structural Formula (1)

   in Structural Formula (1), A is a hydrophilic material, B is a hydrophobic material, X and Y each independently represent a simple covalent bond or a linker-mediated covalent bond, and R represents miR-3139, miR-3189, miR-3199, or miR-8485; or
(iii) nanoparticles including the double-stranded oligonucleotide construct.

Another aspect of the present invention pertains to a cosmetic composition for alleviating hair graying, promoting hair growth, and/or preventing or alleviating hair loss, including, as an active ingredient:
(i) miR-3139, miR-3189, miR-3199, or miR-8485;
(ii) a double-stranded oligonucleotide construct having the structure of Structural Formula (1) below:

   A-X-R-Y-B Structural Formula (1)

   in Structural Formula (1), A is a hydrophilic material, B is a hydrophobic material, X and Y each independently represent a simple covalent bond or a linker-mediated covalent bond, and R represents miR-3139, miR-3189, miR-3199, or miR-8485; or
(iii) nanoparticles including the double-stranded oligonucleotide construct.

In the present invention, the composition is capable of alleviating hair graying, promoting hair growth, and/or preventing or alleviating hair loss by activating melanocytes and proliferating hair follicle cells, but the present invention is not limited thereto.

In the present invention, miR-3139 may include the nucleotide sequence represented by SEQ ID NO: 9 and the nucleotide sequence represented by SEQ ID NO: 10, and is preferably DNA/DNA, RNA/RNA, or a DNA/RNA hybrid composed of the nucleotide sequence represented by SEQ ID NO: 9 and the nucleotide sequence represented by SEQ ID NO: 10.

### miR-3139

5'-CAGGCAUCUGUUGAGCUCCUAUU-3' (SEQ ID NO: 9)
5'-UAGGAGCUCAACAGAUGCCUGUU-3' (SEQ ID NO: 10)

In the present invention, miR-3189 may include the nucleotide sequence represented by SEQ ID NO: 5 and the nucleotide sequence represented by SEQ ID NO: 6, and is preferably DNA/DNA, RNA/RNA, or a DNA/RNA hybrid composed of the nucleotide sequence represented by SEQ ID NO: 5 and the nucleotide sequence represented by SEQ ID NO: 6.

### miR-3189

5'-UGCCCCAUCUGUGCCCUGGGUAGGA-3' (SEQ ID NO: 5)
5'-CCCUUGGGUCUGAUGGGGUAG-3' (SEQ ID NO: 6)

In the present invention, miR-3199 may include the nucleotide sequence represented by SEQ ID NO: 15 and the nucleotide sequence represented by SEQ ID NO: 16, and is preferably DNA/DNA, RNA/RNA, or a DNA/RNA hybrid composed of the nucleotide sequence represented by SEQ ID NO: 15 and the nucleotide sequence represented by SEQ ID NO: 16.

### miR-3199

5'-CUUUCUCCUAAGGCAGUCCCUUU-3' (SEQ ID NO: 15)
5'-AGGGACUGCCUUAGGAGAAAGUU-3' (SEQ ID NO: 16)

In the present invention, miR-8485 may include the nucleotide sequence represented by SEQ ID NO: 1 and the nucleotide sequence represented by SEQ ID NO: 2, and is preferably DNA/DNA, RNA/RNA, or a DNA/RNA hybrid composed of the nucleotide sequence represented by SEQ ID NO: 1 and the nucleotide sequence represented by SEQ ID NO: 2.

### miR-8485

5'-ACGUGUGUGUGUGUGUGUGUU-3' (SEQ ID NO: 1)
5'-CACACACACACACACACGUAU-3' (SEQ ID NO: 2)

In the present invention, miR-3189, miR-3199, and miR-8485 were confirmed to increase expression of microphthalmia-associated transcription factor (MITF), tyrosinase (TYR), tyrosinase-related protein 1 (TYRP1), and tyrosinase-related protein 2 (TYRP2), and miR-3139 was confirmed to increase expression of TYR and TYRP2.

Genes, expression of which is increased by miRNA of the present invention, are known to have the following functions.

MITF, which is a transcription factor, regulates gene expression of TYR, TYRP1, and TYRP2, which are enzymes directly involved in melanin pigment synthesis, and is known as a key transcription factor for melanocyte development and differentiation (D'Mello, S. A., Finlay, G. J., Baguley, B. C. & Askarian-Amiri, M. E. Signaling Pathways in Melanogenesis. Int. J. Mol. Sci. 17 (2016); Kawakami A, Fisher DE. The master role of microphthalmia-associated transcription factor in melanocyte and melanoma biology. Lab. Investig. (2017)).

TYR, TYRP1, and TYRP2 are mainly involved in converting tyrosine into melanin pigment, and in particular, TYR and TYRP2 are known to be important enzymes that affect the quantity and quality of melanin (NIU, C., AISA, H.A., 2017: Upregulation of melanogenesis and tyrosinase activity: potential agents for vitiligo. Molecules 22, E1303. (2017) ; D'Mello, S. A., Finlay, G. J., Baguley, B. C. & Askarian-Amiri, M. E. Signaling Pathways in Melanogenesis. Int. J. Mol. Sci. 17 (2016)).

As described in the background art, the seed region ranging from the 2^{nd} base to the 8^{th} to 9^{th} bases in the active sequence of miRNA is the main factor for activity. When producing a double-stranded oligonucleotide, a long double-stranded oligonucleotide including the same may be constructed and used.

Also, miRNA may be double-stranded, and in another embodiment may include a single-molecule polynucleotide. For example, it may be an antisense oligonucleotide or miRNA, but is not limited thereto.

Meanwhile, miR-3139, miR-3189, miR-3199, or miR-8485 may include a sequence in which at least one base is substituted, deleted, or inserted in a sense strand that makes up the same or an antisense strand complementary thereto.

The double-stranded oligonucleotide according to the present invention may include an overhang, which is a structure including one or more unpaired nucleotides at the 3' end of one or both strands. Also, the sense strand or antisense strand is preferably composed of 19 to 31 nucleotides, but is not limited thereto.

In the present invention, for an oligo conjugate in which a hydrophilic material and a hydrophobic material bind to the RNA or DNA oligonucleotide, the oligonucleotide may be efficiently delivered *in vivo* and may be increased in stability through a conjugate in which a hydrophilic material and a hydrophobic material are conjugated to both ends of the RNA or DNA oligonucleotide.

Moreover, self-assembled nanoparticles are formed by hydrophobic interaction of hydrophobic materials, and such nanoparticles have vastly superior *in-vivo* delivery efficiency and stability, and have a very uniform particle size through structural improvement, such that a process for manufacturing a drug is simple because QC (quality control) is easy.

In an embodiment of the present invention, the hydrophilic material is represented by (P)ₙ, (Pₘ-J)ₙ, or (J-Pₘ)ₙ, in which P is a hydrophilic material monomer, n is 1 to 200, m is 1 to 15, and J is a linker connecting m hydrophilic material monomers to each other or connecting m hydrophilic material monomers and oligonucleotides to each other.

In the present invention, the hydrophilic material may have a molecular weight of 200 to 10,000.

In the present invention, the hydrophilic material may be any one selected from the group consisting of polyethylene glycol, polyvinylpyrrolidone, and polyoxazoline, but is not limited thereto.

In the present invention, the hydrophilic material monomer P may have the structure of Compound (1) below:

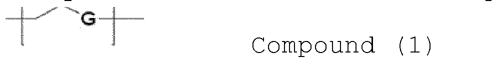

In Compound (1), G is selected from the group consisting of CH₂, O, S, and NH.

In the present invention, the linker J may be selected from the group consisting of PO₃⁻, SO₃, and CO₂.

In the present invention, the hydrophobic material may have a molecular weight of 250 to 1,000, and the hydrophobic material may be selected from the group consisting of a steroid derivative, a glyceride derivative, glycerol ether, polypropylene glycol, a C₁₂-C₅₀ unsaturated or saturated hydrocarbon, diacylphosphatidylcholine, fatty acid, phospholipid, and lipopolyamine, but is not limited thereto.

In the present invention, the steroid derivative may be selected from the group consisting of cholesterol, cholestanol, cholic acid, cholesteryl formate, cholestanyl formate, and cholesteryl amine, but is not limited thereto.

In the present invention, the glyceride derivative may be selected from the group consisting of mono-, di-, and tri-glycerides, but is not limited thereto.

In the present invention, the covalent bond represented by X and Y may be either a non-degradable bond or a degradable bond. Here, the non-degradable bond may be an amide bond or a phosphate bond, and the degradable bond may be a disulfide bond, an acid-degradable bond, an ester bond, an anhydride bond, a biodegradable bond, or an enzymedegradable bond, but the present invention is not limited thereto.

When the hydrophilic material is A, the double-stranded oligonucleotide construct according to the present invention may have the structure of Structural Formula (1') below.

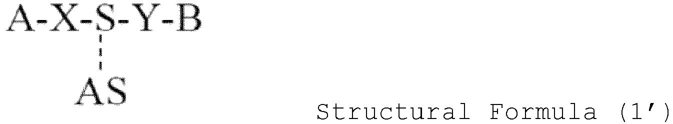

In Structural Formula (1'), A, B, X, and Y are as defined in Structural Formula (1), S represents the sense strand of miRNA, and AS represents the antisense strand of miRNA.

In an embodiment of the present invention, the double-stranded oligonucleotide construct including miRNA according to the present invention may be a double-stranded oligonucleotide construct having the structure of Structural Formula (2) below.

A-X-5' R 3' Y-B Structural Formula (2)

In Structural Formula (2), A, B, X, Y, and R are as defined in Structural Formula 1.

More preferably, the double-stranded oligonucleotide construct has the structure of Structural Formula (2') below.

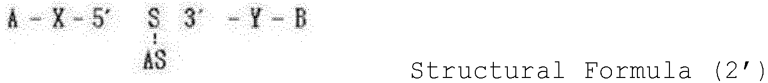

In an embodiment of the present invention, the hydrophilic material may be a cationic or nonionic polymeric material having a molecular weight of 200 to 10,000, preferably a nonionic polymeric material having a molecular weight of 1,000 to 2,000. The hydrophilic material may be a nonionic hydrophilic polymer compound, and may be any one selected from the group consisting of, for example, polyethylene glycol, polyvinylpyrrolidone, and polyoxazoline, but is not limited thereto.

In another embodiment of the present invention, when the hydrophilic material is (Pₘ-J)ₙ or (J-Pₘ)ₙ, the double-stranded oligonucleotide construct according to the present invention has the structure of Structural Formula (3) or Structural Formula (4) below.

(Pₘ-J)ₙ-X-R-Y-B Structural Formula (3)

(J-Pₘ)ₙ-X-R-Y-B Structural Formula (4)

In Structural Formula (3) and Structural Formula (4), P is a hydrophilic material monomer, n is 1 to 200, m is 1 to 15, J is a linker connecting m hydrophilic material monomers to each other or connecting m hydrophilic material monomers and oligonucleotides to each other, X and Y each independently represent a simple covalent bond or a linker-mediated covalent bond, and R represents specific miRNA of the present invention. More preferably, the double-stranded oligonucleotide construct including miRNA according to the present invention has the structure of Structural Formula (3') below.

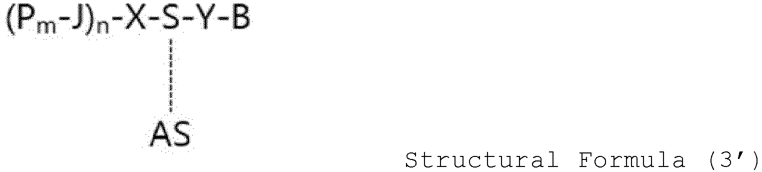

In Structural Formula (3'), P, B, J, m, n, X, and Y are as defined in Structural Formula (3), S represents the sense strand of miRNA, and AS represents the antisense strand of miRNA.

More preferably, the double-stranded oligonucleotide construct including miRNA according to the present invention has the structure of Structural Formula (4') below.

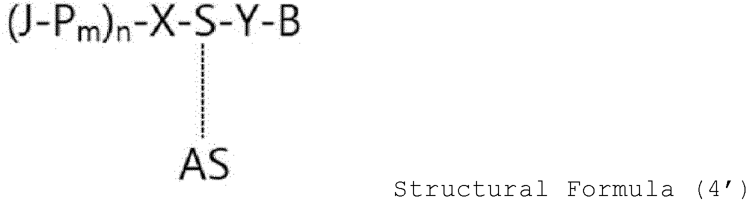

In Structural Formula (4'), P, B, J, m, n, X, and Y are as defined in Structural Formula (4), S represents the sense strand of miRNA, and AS represents the antisense strand of miRNA.

As the hydrophilic material monomer P in Structural Formula (3) and Structural Formula (4), any nonionic hydrophilic polymer monomer may be used without limitation so long as it meets the purpose of the present invention. Preferably a monomer selected from among Compound (1) to Compound (3) shown in Table 1 below, more preferably a monomer of Compound (1), is used, and G in Compound (1) is preferably selected from among CH₂, O, S, and NH.

In particular, among hydrophilic material monomers, the monomer represented by Compound (1) may include various functional groups introduced thereto, may have superior biocompatibility such as good *in-vivo* affinity and low immune response, and may increase *in-vivo* stability of the oligonucleotide contained in the construct according to Structural Formula (3) and Structural Formula (4), and also delivery efficiency thereof, so it is very suitable for the manufacture of the construct according to the present invention.

**[Table 1] Preferred hydrophilic material monomer structure in the present invention**

| Compound (1) | Compound (2) | Compound (3) |
|---|---|---|
| 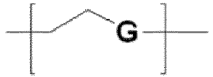 G is CH₂, O, S, or NH | 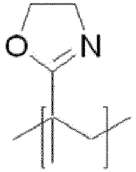 | 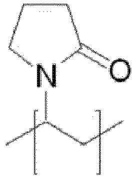 |

It is preferable for the hydrophilic material in Structural Formula (3) and Structural Formula (4) to have a total molecular weight in the range of 1,000 to 2,000. Therefore, for example, when hexaethylene glycol according to Compound (1), namely a material in which G is O and m is 6 in Structural Formula (3) and Structural Formula (4), is used, the molecular weight of the hexaethylene glycol spacer is 344, so the number of repetitions n is preferably 3 to 5. The present invention is characterized in that the repeat unit of the hydrophilic group, represented as (Pₘ-J) or (J-Pₘ) in Structural Formula (3) and Structural Formula (4), namely a hydrophilic material block, may be used in an appropriate number, represented by n, as necessary. The hydrophilic material monomer P and the linker J included in each of the hydrophilic material blocks may be independently the same or different in the hydrophilic material blocks. Specifically, when three hydrophilic material blocks are used (n=3), the first block may include the hydrophilic material monomer according to Compound (1), the second block may include the hydrophilic material monomer according to Compound (2), and the third block may include the hydrophilic material monomer according to Compound (3). In this way, different hydrophilic material monomers may be used for all hydrophilic material blocks, or any one hydrophilic material monomer selected from among the hydrophilic material monomers according to Compound (1) to Compound (3) may be identically used for all hydrophilic material blocks. Likewise, the linker that mediates binding of the hydrophilic material monomers may also use the same linker for hydrophilic material blocks or different linkers for hydrophilic material blocks. In addition, m, which is the number of hydrophilic material monomers, may be the same or different in the hydrophilic material blocks. Specifically, three hydrophilic material monomers (m=3) may be connected in the first hydrophilic material block, five hydrophilic material monomers (m=5) may be connected in the second hydrophilic material block, and four hydrophilic material monomers (m=4) may be connected in the third hydrophilic material block. In this way, different numbers of hydrophilic material monomers may be used, or the same number of hydrophilic material monomers may be used in all hydrophilic material blocks.

In the present invention, the linker J is preferably selected from the group consisting of PO₃⁻, SO₃, and CO₂, but is not limited thereto. Any linker may be used, so long as it meets the purpose of the present invention depending on the monomer of the hydrophilic material that is used, as will be obvious to those of ordinary skill in the art.

All or part of the hydrophilic material monomers may be modified to have a functional group required for binding to other materials such as a target-specific ligand, as necessary.

In some cases, one to three phosphate groups may bind to the 5' end of the antisense strand of the double-stranded oligonucleotide construct including the genespecific miRNA.

For example, the double-stranded oligonucleotide construct including miRNA has the structure of Structural Formula (3") or Structural Formula (4") below.

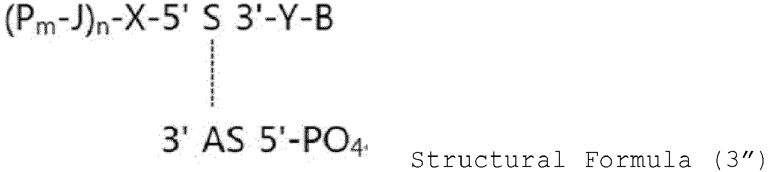

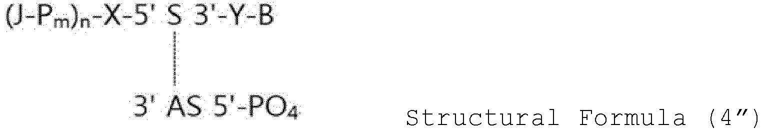

The hydrophobic material B serves to form nanoparticles composed of the oligonucleotide construct according to Structural Formula (1) through hydrophobic interaction.

The hydrophobic material preferably has a molecular weight of 250 to 1,000, and examples thereof may include, but are not limited to, a steroid derivative, a glyceride derivative, glycerol ether, polypropylene glycol, a C₁₂-C₅₀ unsaturated or saturated hydrocarbon, diacylphosphatidylcholine, fatty acid, phospholipid, lipopolyamine, and the like, and any hydrophobic material may be used so long as it meets the purpose of the present invention, as will be obvious to those of ordinary skill in the art to which the present invention belongs.

The steroid derivative may be selected from the group consisting of cholesterol, cholestanol, cholic acid, cholesteryl formate, cholestanyl formate, and cholesteryl amine, and the glyceride derivative may be selected from the group consisting of mono-, di- and tri-glycerides. Here, fatty acid of the glyceride is preferably a C₁₂-C₅₀ unsaturated or saturated fatty acid.

In particular, among hydrophobic materials, saturated or unsaturated hydrocarbon or cholesterol is preferred in that it has the advantage of being able to easily bind during synthesis of the oligonucleotide construct according to the present invention.

The hydrophobic material may bind to the distal end of the hydrophilic material, and may bind to any position on the sense strand or antisense strand of miRNA.

In the present invention, the hydrophilic material, the hydrophilic material block, or the hydrophobic material and the oligonucleotide are linked via a simple covalent bond or a linker-mediated covalent bond (X or Y). The covalent bond may be either a non-degradable bond or a degradable bond. Here, the non-degradable bond may be an amide bond or a phosphate bond, and the degradable bond may be a disulfide bond, an acid-degradable bond, an ester bond, an anhydride bond, a biodegradable bond, or an enzymedegradable bond, but the present invention is not limited thereto.

The miRNA sequence that may be used as the active ingredient of the composition for alleviating hair graying, preventing or alleviating hair loss, promoting hair follicle cell proliferation, and/or promoting hair growth by activating melanocytes and promoting melanogenesis, according to the present invention, may include human genome-derived sequences or the miRNA-derived genome is not limited to the human genome, and miRNA sequences obtained from other animal-derived genomes may also be used.

The miRNA may be used in any miRNA mimic form that generates bioequivalent efficacy of miRNA, and modified miRNA including the miRNA sequence containing the same seed region may be used. Here, the length of the sense and antisense sequences of miRNA may be reduced, and a short mimic 15 nucleotides in length may also be used.

The miRNA mimic for miRNA may partially include a phosphorothioate structure, which is a form in which the RNA phosphate backbone structure is substituted with another element such as sulfur, and may be used in a form in which RNA is fully or partially substituted with DNA, RNA (peptide nucleic acid), or LNA (locked nucleic acid) molecules. Moreover, it may be used in a form in which the 2'-hydroxyl group of RNA sugar is substituted with various functional structures, which include, but are not limited to, methylation, methoxylation, fluorination, etc.

The miRNA is not limited to double-stranded RNA of mature miRNA and the miRNA mimic derived therefrom, and may be used in the form of a miRNA precursor. The miRNA precursor may also include the above-described RNA phosphate backbone structure, partial or full substitution of RNA with DNA, PNA, LNA, etc., modification of the 2' hydroxyl group of the RNA sugar molecule, and the like.

The miRNA may be used in the form of a miRNA precursor or primary miRNA (pre-miRNA), which may be chemically synthesized or delivered in the form of a plasmid to cells and thus expressed.

In the present invention, miRNA may be delivered to cells cultured in a culture dish through methods of using a mixture with cationic lipids, by electrical stimulation, or using a virus. Various methods known in the art for miRNA delivery may be easily performed by those skilled in the art, and the present invention is not limited thereto.

Still another aspect of the present invention pertains to a double-stranded oligonucleotide construct including miRNA such as miR-3139, miR-3189, miR-3199, or miR-8485 and to nanoparticles including the same.

As described above, the double-stranded oligonucleotide construct including miRNA is amphiphilic, containing both hydrophobic and hydrophilic materials, and the hydrophilic portion is directed outward due to affinity through interactions such as hydrogen bonds, etc. with water molecules present in the body and hydrophobic materials are directed inward through hydrophobic interaction therebetween to form thermodynamically stable nanoparticles. Specifically, nanoparticles are provided to protect the miRNA sequence by positioning a hydrophobic material at the center of the nanoparticles and a hydrophilic material outside the double-stranded oligonucleotide including miRNA.

The nanoparticles according to the present invention may be formed of only a double-stranded oligonucleotide construct having the same sequence or may be composed of double-stranded oligonucleotide constructs having different sequences, and double-stranded oligonucleotide constructs including different miRNAs targeting melanogenesis-stimulating and hair follicle cell proliferation-stimulating genes may be included in the nanoparticles according to the present invention. For example, the nanoparticles may be formed by mixing a double-stranded oligonucleotide construct including miR-3139 and a double-stranded oligonucleotide construct including miR-8485.

Also, the composition according to the present invention may further include, in addition to the double-stranded oligonucleotide construct including miRNA, a double-stranded oligonucleotide including miRNA that activates melanocytes and promotes melanogenesis and hair follicle cell proliferation, or a double-stranded oligonucleotide construct including the same.

In the present invention, it was confirmed that the double-stranded oligonucleotide construct (SAMiRNA^{™}) including miRNA and the nanoparticles were effective at activating melanocytes and promoting melanogenesis, thereby alleviating hair graying. Moreover, the effect of promoting proliferation of hair follicle dermal papilla cells and keratinocytes was confirmed in addition to melanocyte activation.

Therefore, the present invention is capable of utilizing the composition for pharmaceutical and cosmetic application, in which the pharmaceutical composition may be used in any formulation selected from among formulations such as ointments, pastes, gels, jellies, serums, aerosol sprays, non-aerosol sprays, foams, creams, lotions, solutions, and suspensions, but is not limited thereto.

When the composition is used as a cosmetic, it may be used in any formulation selected from among formulations such as hair tonics, hair conditioners, hair essences, hair lotions, hair nutrition lotions, hair shampoos, hair rinses, hair treatments, hair creams, hair nutrition creams, hair moisture creams, hair massage creams, hair waxes, hair aerosols, hair packs, hair nutrition packs, hair soaps, hair cleansing foams, hair oils, hair drying agents, hair preservatives, hair dyes, hair waving agents, hair bleaches, hair gels, hair glazes, hair dressings, hair lacquers, hair moisturizers, hair mousses, and hair sprays, but is not limited thereto.

The composition of the present invention may be produced by including at least one pharmaceutically acceptable carrier in addition to the above active ingredient. Such a pharmaceutically acceptable carrier has to be compatible with the active ingredient of the present invention, and may include saline, sterile water, Ringer's solution, buffered saline, dextrose solution, maltodextrin solution, glycerol, and ethanol, which may be used alone or in combination of two or more thereof, and as necessary, other typical additives such as antioxidants, buffers, and bacteriostatic agents may be added. Also, diluents, dispersants, surfactants, binders, and lubricants may be further added to form an injectable formulation such as an aqueous solution, suspension, emulsion, and the like. In particular, it is preferable to provide a formulation in a lyophilized form. For preparation of the lyophilized formulation, a method commonly known in the art to which the present invention belongs may be used, and a stabilizer for lyophilization may be added. Moreover, the composition is preferably formulated depending on each disease or ingredient using a method disclosed in Remington's Pharmaceutical Science (Mack Publishing Company, Easton PA) as an appropriate method in the art.

The amount and administration mode of the active ingredient, etc. included in the composition of the present invention may be determined by those skilled in the art based on the number of melanocytes, the extent of functional decline, graying symptoms, and the severity of graying site of an ordinary individual. Moreover, the composition may be formulated in various forms such as powders, tablets, injections, ointments, and functional cosmetics, and may be provided in unit-dose or multi-dose containers, such as sealed ampoules and bottles.

When the double-stranded oligonucleotide construct including miRNA according to the present invention, and the composition or nanoparticles including the same are used for the manufacture of functional cosmetics or topical skin preparations, the formulation of functional cosmetics or topical skin preparations may be selected from the group consisting of creams, lotions, gels, water-soluble liquids, and essences, without being limited thereto.

Yet another aspect of the present invention pertains to a method of alleviating hair graying, promoting hair growth, and/or preventing or alleviating hair loss including administering, to a subject in need of alleviating hair graying, promoting hair growth, and/or preventing or alleviating hair loss,
(i) miR-3139, miR-3189, miR-3199, or miR-8485;
(ii) a double-stranded oligonucleotide construct having the structure of Structural Formula (1) below:

   A-X-R-Y-B Structural Formula (1)

   in Structural Formula (1), A is a hydrophilic material, B is a hydrophobic material, X and Y each independently represent a simple covalent bond or a linker-mediated covalent bond, and R represents miR-3139, miR-3189, miR-3199, or miR-8485; or
(iii) nanoparticles including the double-stranded oligonucleotide construct.

A further aspect of the present invention pertains to the use of, for alleviating hair graying, promoting hair growth, and/or preventing or alleviating hair loss,
(i) miR-3139, miR-3189, miR-3199, or miR-8485;
(ii) a double-stranded oligonucleotide construct having the structure of Structural Formula (1) below:

   A-X-R-Y-B Structural Formula (1)

   in Structural Formula (1), A is a hydrophilic material, B is a hydrophobic material, X and Y each independently represent a simple covalent bond or a linker-mediated covalent bond, and R represents miR-3139, miR-3189, miR-3199, or miR-8485; or (iii) nanoparticles including the double-stranded oligonucleotide construct.

Still a further aspect of the present invention pertains to the use of, for the manufacture of medicaments or cosmetics for alleviating hair graying, promoting hair growth, and/or preventing or alleviating hair loss, (i) miR-3139, miR-3189, miR-3199, or miR- 8485;
(ii) a double-stranded oligonucleotide construct having the structure of Structural Formula (1) below:

   A-X-R-Y-B Structural Formula (1)
in Structural Formula (1), A is a hydrophilic material, B is a hydrophobic material, X and Y each independently represent a simple covalent bond or a linker-mediated covalent bond, and R represents miR-3139, miR-3189, miR-3199, or miR-8485; or (iii) nanoparticles including the double-stranded oligonucleotide construct.

### Examples

A better understanding of the present invention may be obtained through the following examples. These examples are merely set forth to illustrate the present invention, and are not constructed as limiting the scope of the present invention, as will be apparent to those skilled in the art.

### Example 1: Screening for selection of miRNAs promoting melanogenesis

### 1-1. Candidates for human miRNAs promoting melanogenesis

As 21 versions of human miRNA sequences provided by miRBase (www.mirbase.org), which is a miRNA database, 1728 miRNAs based on the stem-loop structure were synthesized in a form loaded on a double-stranded oligonucleotide construct (SAMiRNA^{™}) (Table 2). All synthesized miRNA strands were identified and confirmed for synthesis of the intended sequence using MALDI-TOF mass spectrometry. Screening was conducted to discover miRNAs with the highest amount of melanin produced using 1728 miRNAs in human skin cancer cell lines. The antisense strand sequences of 1728 human miRNAs tested in the present invention are known on miRbase (http://www.mirbase.org), and when the seed region was provided on only one strand, a sequence complementary thereto was synthesized for delivery in a double-stranded form to cells.

For example, miRNA of miR-3139 was produced as follows.
SEQ ID NO: 9: 5'-CAGGCAUCUGUUGAGCUCCUAUU-3'
SEQ ID NO: 10: 5'-UAGGAGCUCAACAGAUGCCUGUU-3'

Also, when the seed sequence was provided on both strands, respective strands were synthesized as sense and antisense strands. For example, miRNA of miR-3189 was produced as follows.
SEQ ID NO: 5: 5'-UGCCCCAUCUGUGCCCUGGGUAGGA-3' (sense)
SEQ ID NO: 6: 5'-CCCUUGGGUCUGAUGGGGUAG-3' (antisense)

**[Table 2] List of 1728 human miRNAs tested in the present invention**

| miRNA | | |
|---|---|---|
| hsa-let-7a-1 | hsa-miR-1197 | hsa-miR-1249 |
| hsa-let-7a-2 | hsa-miR-1199 | hsa-miR-1250 |
| hsa-let-7b | hsa-miR-1200 | hsa-miR-1251 |
| hsa-let-7c | hsa-miR-1202 | hsa-miR-1252 |
| hsa-let-7d | hsa-miR-1203 | hsa-miR-1253 |
| hsa-let-7e | hsa-miR-1204 | hsa-miR-1254 |
| hsa-let-7f-1 | hsa-miR-1205 | hsa-miR-1255a |
| hsa-let-7f-2 | hsa-miR-1206 | hsa-miR-1255b |
| hsa-let-7g | hsa-miR-1207 | hsa-miR-1256 |
| hsa-let-7i | hsa-miR-1208 | hsa-miR-1257 |
| hsa-miR-1 | hsa-miR-122 | hsa-miR-1258 |
| hsa-miR-100 | hsa-miR-1224 | hsa-miR-125a |
| hsa-miR-101 | hsa-miR-1225 | hsa-miR-125b-1 |
| hsa-miR-103a | hsa-miR-1226 | hsa-miR-125b-2 |
| hsa-miR-103b | hsa-miR-1227 | hsa-miR-126 |
| hsa-miR-105 | hsa-miR-1228 | hsa-miR-1260a |
| hsa-miR-106a | hsa-miR-1229 | hsa-miR-1260b |
| hsa-miR-106b | hsa-miR-1231 | hsa-miR-1261 |
| hsa-miR-107 | hsa-miR-1233 | hsa-miR-1262 |
| hsa-miR-10a | hsa-miR-1234 | hsa-miR-1263 |
| hsa-miR-10b | hsa-miR-1236 | hsa-miR-1264 |
| hsa-miR-1178 | hsa-miR-1237 | hsa-miR-1265 |
| hsa-miR-1179 | hsa-miR-1238 | hsa-miR-1266 |
| hsa-miR-1180 | hsa-miR-124 | hsa-miR-1267 |
| hsa-miR-1181 | hsa-miR-1243 | hsa-miR-1268a |
| hsa-miR-1182 | hsa-miR-1244 | hsa-miR-1268b |
| hsa-miR-1183 | hsa-miR-1245a | hsa-miR-1269a |
| hsa-miR-1184 | hsa-miR-1245b | hsa-miR-1269b |
| hsa-miR-1185-1 | hsa-miR-1246 | hsa-miR-127 |
| hsa-miR-1185-2 | hsa-miR-1247 | hsa-miR-1270 |
| hsa-miR-1193 | hsa-miR-1248 | hsa-miR-1271 |
| hsa-miR-1272 | hsa-miR-1295b | hsa-miR-142 |
| hsa-miR-1273a | hsa-miR-1296 | hsa-miR-143 |
| hsa-miR-1273c | hsa-miR-1297 | hsa-miR-144 |
| hsa-miR-1273d | hsa-miR-1298 | hsa-miR-145 |
| hsa-miR-1273e | hsa-miR-1299 | hsa-miR-1468 |
| hsa-miR-1273f | hsa-miR-1301 | hsa-miR-1469 |
| hsa-miR-1273g | hsa-miR-1302 | hsa-miR-146a |
| hsa-miR-1273h | hsa-miR-1303 | hsa-miR-146b |
| hsa-miR-1275 | hsa-miR-1304 | hsa-miR-1470 |
| hsa-miR-1276 | hsa-miR-1305 | hsa-miR-1471 |
| hsa-miR-1277 | hsa-miR-1306 | hsa-miR-147a |
| hsa-miR-1278 | hsa-miR-1307 | hsa-miR-147b |
| hsa-miR-1279 | hsa-miR-130a | hsa-miR-148a |
| hsa-miR-128-1 | hsa-miR-130b | hsa-miR-148b |
| hsa-miR-128-2 | hsa-miR-132 | hsa-miR-149 |
| hsa-miR-1281 | hsa-miR-1321 | hsa-miR-150 |
| hsa-miR-1282 | hsa-miR-1322 | hsa-miR-151a |
| hsa-miR-1283 | hsa-miR-1323 | hsa-miR-151b |
| hsa-miR-1284 | hsa-miR-1324 | hsa-miR-152 |
| hsa-miR-1285 | hsa-miR-133a | hsa-miR-153 |
| hsa-miR-1286 | hsa-miR-133b | hsa-miR-1537 |
| hsa-miR-1287 | hsa-miR-134 | hsa-miR-1538 |
| hsa-miR-1288 | hsa-miR-1343 | hsa-miR-1539 |
| hsa-miR-1289 | hsa-miR-135a | hsa-miR-154 |
| hsa-miR-129-1 | hsa-miR-135b | hsa-miR-155 |
| hsa-miR-129-2 | hsa-miR-136 | hsa-miR-1587 |
| hsa-miR-1290 | hsa-miR-137 | hsa-miR-15a |
| hsa-miR-1291 | hsa-miR-138-1 | hsa-miR-15b |
| hsa-miR-1292 | hsa-miR-138-2 | hsa-miR-16-1 |
| hsa-miR-1293 | hsa-miR-139 | hsa-miR-16-2 |
| hsa-miR-1294 | hsa-miR-140 | hsa-miR-17 |
| hsa-miR-1295a | hsa-miR-141 | hsa-miR-181a-1 |
| hsa-miR-181a-2 | hsa-miR-196a | hsa-miR-2113 |
| hsa-miR-181b-1 | hsa-miR-196b | hsa-miR-2114 |
| hsa-miR-181b-2 | hsa-miR-197 | hsa-miR-2115 |
| hsa-miR-181c | hsa-miR-1972 | hsa-miR-2116 |
| hsa-miR-181d | hsa-miR-1973 | hsa-miR-2117 |
| hsa-miR-182 | hsa-miR-1976 | hsa-miR-212 |
| hsa-miR-1825 | hsa-miR-198 | hsa-miR-214 |
| hsa-miR-1827 | hsa-miR-199a | hsa-miR-215 |
| hsa-miR-183 | hsa-miR-199b | hsa-miR-216a |
| hsa-miR-184 | hsa-miR-19a | hsa-miR-216b |
| hsa-miR-185 | hsa-miR-19b-1 | hsa-miR-217 |
| hsa-miR-186 | hsa-miR-19b-2 | hsa-miR-218-1 |
| hsa-miR-187 | hsa-miR-200a | hsa-miR-218-2 |
| hsa-miR-188 | hsa-miR-200b | hsa-miR-219a-1 |
| hsa-miR-18a | hsa-miR-200c | hsa-miR-219a-2 |
| hsa-miR-18b | hsa-miR-202 | hsa-miR-219b |
| hsa-miR-1908 | hsa-miR-203a | hsa-miR-22 |
| hsa-miR-1909 | hsa-miR-203b | hsa-miR-221 |
| hsa-miR-190a | hsa-miR-204 | hsa-miR-222 |
| hsa-miR-190b | hsa-miR-205 | hsa-miR-223 |
| hsa-miR-191 | hsa-miR-2052 | hsa-miR-224 |
| hsa-miR-1910 | hsa-miR-2053 | hsa-miR-2276 |
| hsa-miR-1911 | hsa-miR-2054 | hsa-miR-2277 |
| hsa-miR-1912 | hsa-miR-206 | hsa-miR-2278 |
| hsa-miR-1913 | hsa-miR-208a | hsa-miR-2355 |
| hsa-miR-1914 | hsa-miR-208b | hsa-miR-2392 |
| hsa-miR-1915 | hsa-miR-20a | hsa-miR-23a |
| hsa-miR-192 | hsa-miR-20b | hsa-miR-23b |
| hsa-miR-193a | hsa-miR-21 | hsa-miR-23c |
| hsa-miR-193b | hsa-miR-210 | hsa-miR-24-1 |
| hsa-miR-194 | hsa-miR-211 | hsa-miR-24-2 |
| hsa-miR-195 | hsa-miR-2110 | hsa-miR-2467 |
| hsa-miR-25 | hsa-miR-30b | hsa-miR-3140 |
| hsa-miR-2681 | hsa-miR-30c-1 | hsa-miR-3141 |
| hsa-miR-2682 | hsa-miR-30c-2 | hsa-miR-3142 |
| hsa-miR-26a-1 | hsa-miR-30d | hsa-miR-3143 |
| hsa-miR-26a-2 | hsa-miR-30e | hsa-miR-3144 |
| hsa-miR-26b | hsa-miR-31 | hsa-miR-3145 |
| hsa-miR-27a | hsa-miR-3115 | hsa-miR-3146 |
| hsa-miR-27b | hsa-miR-3116 | hsa-miR-3147 |
| hsa-miR-28 | hsa-miR-3117 | hsa-miR-3148 |
| hsa-miR-2861 | hsa-miR-3118 | hsa-miR-3149 |
| hsa-miR-2909 | hsa-miR-3119 | hsa-miR-3150a |
| hsa-miR-296 | hsa-miR-3120 | hsa-miR-3150b |
| hsa-miR-297 | hsa-miR-3121 | hsa-miR-3151 |
| hsa-miR-298 | hsa-miR-3122 | hsa-miR-3152 |
| hsa-miR-299 | hsa-miR-3123 | hsa-miR-3153 |
| hsa-miR-29a | hsa-miR-3124 | hsa-miR-3154 |
| hsa-miR-29b-1 | hsa-miR-3125 | hsa-miR-3155a |
| hsa-miR-29b-2 | hsa-miR-3126 | hsa-miR-3155b |
| hsa-miR-29c | hsa-miR-3127 | hsa-miR-3156 |
| hsa-miR-300 | hsa-miR-3128 | hsa-miR-3157 |
| hsa-miR-301a | hsa-miR-3129 | hsa-miR-3158 |
| hsa-miR-301b | hsa-miR-3130 | hsa-miR-3159 |
| hsa-miR-302a | hsa-miR-3131 | hsa-miR-3160 |
| hsa-miR-302b | hsa-miR-3132 | hsa-miR-3161 |
| hsa-miR-302c | hsa-miR-3133 | hsa-miR-3162 |
| hsa-miR-302d | hsa-miR-3134 | hsa-miR-3163 |
| hsa-miR-302e | hsa-miR-3135a | hsa-miR-3164 |
| hsa-miR-302f | hsa-miR-3135b | hsa-miR-3165 |
| hsa-miR-3064 | hsa-miR-3136 | hsa-miR-3166 |
| hsa-miR-3065 | hsa-miR-3137 | hsa-miR-3167 |
| hsa-miR-3074 | hsa-miR-3138 | hsa-miR-3168 |
| hsa-miR-30a | hsa-miR-3139 | hsa-miR-3169 |
| hsa-miR-3170 | hsa-miR-3202 | hsa-miR-3607 |
| hsa-miR-3171 | hsa-miR-320a | hsa-miR-3609 |
| hsa-miR-3173 | hsa-miR-320b | hsa-miR-361 |
| hsa-miR-3174 | hsa-miR-320c | hsa-miR-3610 |
| hsa-miR-3175 | hsa-miR-320d | hsa-miR-3611 |
| hsa-miR-3176 | hsa-miR-320e | hsa-miR-3612 |
| hsa-miR-3177 | hsa-miR-323a | hsa-miR-3613 |
| hsa-miR-3178 | hsa-miR-323b | hsa-miR-3614 |
| hsa-miR-3179 | hsa-miR-324 | hsa-miR-3615 |
| hsa-miR-3180 | hsa-miR-325 | hsa-miR-3616 |
| hsa-miR-3181 | hsa-miR-326 | hsa-miR-3617 |
| hsa-miR-3182 | hsa-miR-328 | hsa-miR-3618 |
| hsa-miR-3183 | hsa-miR-329 | hsa-miR-3619 |
| hsa-miR-3184 | hsa-miR-330 | hsa-miR-362 |
| hsa-miR-3185 | hsa-miR-331 | hsa-miR-3620 |
| hsa-miR-3186 | hsa-miR-335 | hsa-miR-3621 |
| hsa-miR-3187 | hsa-miR-337 | hsa-miR-3622a |
| hsa-miR-3188 | hsa-miR-338 | hsa-miR-3622b |
| hsa-miR-3189 | hsa-miR-339 | hsa-miR-363 |
| hsa-miR-3190 | hsa-miR-33a | hsa-miR-3646 |
| hsa-miR-3191 | hsa-miR-33b | hsa-miR-3648 |
| hsa-miR-3192 | hsa-miR-340 | hsa-miR-3649 |
| hsa-miR-3193 | hsa-miR-342 | hsa-miR-3650 |
| hsa-miR-3194 | hsa-miR-345 | hsa-miR-3651 |
| hsa-miR-3195 | hsa-miR-346 | hsa-miR-3652 |
| hsa-miR-3196 | hsa-miR-34a | hsa-miR-3653 |
| hsa-miR-3197 | hsa-miR-34b | hsa-miR-3654 |
| hsa-miR-3198 | hsa-miR-34c | hsa-miR-3655 |
| hsa-miR-3199 | hsa-miR-3529 | hsa-miR-3656 |
| hsa-miR-32 | hsa-miR-3591 | hsa-miR-3657 |
| hsa-miR-3200 | hsa-miR-3605 | hsa-miR-3658 |
| hsa-miR-3201 | hsa-miR-3606 | hsa-miR-3659 |
| hsa-miR-365a | hsa-miR-3689d | hsa-miR-378j |
| hsa-miR-365b | hsa-miR-3689e | hsa-miR-379 |
| hsa-miR-3660 | hsa-miR-3689f | hsa-miR-380 |
| hsa-miR-3661 | hsa-miR-369 | hsa-miR-381 |
| hsa-miR-3662 | hsa-miR-3690 | hsa-miR-382 |
| hsa-miR-3663 | hsa-miR-3691 | hsa-miR-383 |
| hsa-miR-3664 | hsa-miR-3692 | hsa-miR-384 |
| hsa-miR-3665 | hsa-miR-370 | hsa-miR-3907 |
| hsa-miR-3666 | hsa-miR-3713 | hsa-miR-3908 |
| hsa-miR-3667 | hsa-miR-3714 | hsa-miR-3909 |
| hsa-miR-3668 | hsa-miR-371a | hsa-miR-3910 |
| hsa-miR-367 | hsa-miR-371b | hsa-miR-3911 |
| hsa-miR-3670 | hsa-miR-372 | hsa-miR-3912 |
| hsa-miR-3671 | hsa-miR-373 | hsa-miR-3913 |
| hsa-miR-3672 | hsa-miR-374a | hsa-miR-3914 |
| hsa-miR-3674 | hsa-miR-374b | hsa-miR-3915 |
| hsa-miR-3675 | hsa-miR-374c | hsa-miR-3916 |
| hsa-miR-3677 | hsa-miR-375 | hsa-miR-3917 |
| hsa-miR-3678 | hsa-miR-376a-1 | hsa-miR-3918 |
| hsa-miR-3679 | hsa-miR-376a-2 | hsa-miR-3919 |
| hsa-miR-3680 | hsa-miR-376b | hsa-miR-3920 |
| hsa-miR-3681 | hsa-miR-376c | hsa-miR-3921 |
| hsa-miR-3682 | hsa-miR-377 | hsa-miR-3922 |
| hsa-miR-3683 | hsa-miR-378a | hsa-miR-3923 |
| hsa-miR-3684 | hsa-miR-378b | hsa-miR-3924 |
| hsa-miR-3685 | hsa-miR-378c | hsa-miR-3925 |
| hsa-miR-3686 | hsa-miR-378d | hsa-miR-3926 |
| hsa-miR-3687 | hsa-miR-378e | hsa-miR-3927 |
| hsa-miR-3688 | hsa-miR-378f | hsa-miR-3928 |
| hsa-miR-3689a | hsa-miR-378g | hsa-miR-3929 |
| hsa-miR-3689b | hsa-miR-378h | hsa-miR-3934 |
| hsa-miR-3689c | hsa-miR-378i | hsa-miR-3935 |
| hsa-miR-3936 | hsa-miR-4256 | hsa-miR-4288 |
| hsa-miR-3937 | hsa-miR-4257 | hsa-miR-4289 |
| hsa-miR-3938 | hsa-miR-4258 | hsa-miR-429 |
| hsa-miR-3939 | hsa-miR-4259 | hsa-miR-4290 |
| hsa-miR-3940 | hsa-miR-4260 | hsa-miR-4291 |
| hsa-miR-3941 | hsa-miR-4261 | hsa-miR-4292 |
| hsa-miR-3942 | hsa-miR-4262 | hsa-miR-4293 |
| hsa-miR-3943 | hsa-miR-4263 | hsa-miR-4294 |
| hsa-miR-3944 | hsa-miR-4264 | hsa-miR-4295 |
| hsa-miR-3945 | hsa-miR-4265 | hsa-miR-4296 |
| hsa-miR-3960 | hsa-miR-4266 | hsa-miR-4297 |
| hsa-miR-3972 | hsa-miR-4267 | hsa-miR-4298 |
| hsa-miR-3973 | hsa-miR-4268 | hsa-miR-4299 |
| hsa-miR-3974 | hsa-miR-4269 | hsa-miR-4300 |
| hsa-miR-3975 | hsa-miR-4270 | hsa-miR-4301 |
| hsa-miR-3976 | hsa-miR-4271 | hsa-miR-4302 |
| hsa-miR-3977 | hsa-miR-4272 | hsa-miR-4303 |
| hsa-miR-3978 | hsa-miR-4273 | hsa-miR-4304 |
| hsa-miR-409 | hsa-miR-4274 | hsa-miR-4305 |
| hsa-miR-410 | hsa-miR-4275 | hsa-miR-4306 |
| hsa-miR-411 | hsa-miR-4276 | hsa-miR-4307 |
| hsa-miR-412 | hsa-miR-4277 | hsa-miR-4308 |
| hsa-miR-421 | hsa-miR-4278 | hsa-miR-4309 |
| hsa-miR-422a | hsa-miR-4279 | hsa-miR-431 |
| hsa-miR-423 | hsa-miR-4280 | hsa-miR-4310 |
| hsa-miR-424 | hsa-miR-4281 | hsa-miR-4311 |
| hsa-miR-425 | hsa-miR-4282 | hsa-miR-4312 |
| hsa-miR-4251 | hsa-miR-4283 | hsa-miR-4313 |
| hsa-miR-4252 | hsa-miR-4284 | hsa-miR-4314 |
| hsa-miR-4253 | hsa-miR-4285 | hsa-miR-4315 |
| hsa-miR-4254 | hsa-miR-4286 | hsa-miR-4316 |
| hsa-miR-4255 | hsa-miR-4287 | hsa-miR-4317 |
| hsa-miR-4318 | hsa-miR-4433a | hsa-miR-4463 |
| hsa-miR-4319 | hsa-miR-4433b | hsa-miR-4464 |
| hsa-miR-432 | hsa-miR-4434 | hsa-miR-4465 |
| hsa-miR-4320 | hsa-miR-4435 | hsa-miR-4466 |
| hsa-miR-4321 | hsa-miR-4436a | hsa-miR-4467 |
| hsa-miR-4322 | hsa-miR-4436b | hsa-miR-4468 |
| hsa-miR-4323 | hsa-miR-4437 | hsa-miR-4469 |
| hsa-miR-4324 | hsa-miR-4438 | hsa-miR-4470 |
| hsa-miR-4325 | hsa-miR-4439 | hsa-miR-4471 |
| hsa-miR-4326 | hsa-miR-4440 | hsa-miR-4472 |
| hsa-miR-4327 | hsa-miR-4441 | hsa-miR-4473 |
| hsa-miR-4328 | hsa-miR-4442 | hsa-miR-4474 |
| hsa-miR-4329 | hsa-miR-4443 | hsa-miR-4475 |
| hsa-miR-433 | hsa-miR-4444 | hsa-miR-4476 |
| hsa-miR-4330 | hsa-miR-4445 | hsa-miR-4477a |
| hsa-miR-4417 | hsa-miR-4446 | hsa-miR-4477b |
| hsa-miR-4418 | hsa-miR-4447 | hsa-miR-4478 |
| hsa-miR-4419a | hsa-miR-4448 | hsa-miR-4479 |
| hsa-miR-4419b | hsa-miR-4449 | hsa-miR-448 |
| hsa-miR-4420 | hsa-miR-4450 | hsa-miR-4480 |
| hsa-miR-4421 | hsa-miR-4451 | hsa-miR-4481 |
| hsa-miR-4422 | hsa-miR-4452 | hsa-miR-4482 |
| hsa-miR-4423 | hsa-miR-4453 | hsa-miR-4483 |
| hsa-miR-4424 | hsa-miR-4454 | hsa-miR-4484 |
| hsa-miR-4425 | hsa-miR-4455 | hsa-miR-4485 |
| hsa-miR-4426 | hsa-miR-4456 | hsa-miR-4486 |
| hsa-miR-4427 | hsa-miR-4457 | hsa-miR-4487 |
| hsa-miR-4428 | hsa-miR-4458 | hsa-miR-4488 |
| hsa-miR-4429 | hsa-miR-4459 | hsa-miR-4489 |
| hsa-miR-4430 | hsa-miR-4460 | hsa-miR-4490 |
| hsa-miR-4431 | hsa-miR-4461 | hsa-miR-4491 |
| hsa-miR-4432 | hsa-miR-4462 | hsa-miR-4492 |
| hsa-miR-4493 | hsa-miR-4519 | hsa-miR-4635 |
| hsa-miR-4494 | hsa-miR-451a | hsa-miR-4636 |
| hsa-miR-4495 | hsa-miR-451b | hsa-miR-4637 |
| hsa-miR-4496 | hsa-miR-452 | hsa-miR-4638 |
| hsa-miR-4497 | hsa-miR-4520-1 | hsa-miR-4639 |
| hsa-miR-4498 | hsa-miR-4520-2 | hsa-miR-4640 |
| hsa-miR-4499 | hsa-miR-4521 | hsa-miR-4641 |
| hsa-miR-449a | hsa-miR-4522 | hsa-miR-4642 |
| hsa-miR-449b | hsa-miR-4523 | hsa-miR-4643 |
| hsa-miR-449c | hsa-miR-4524a | hsa-miR-4644 |
| hsa-miR-4500 | hsa-miR-4524b | hsa-miR-4645 |
| hsa-miR-4501 | hsa-miR-4525 | hsa-miR-4646 |
| hsa-miR-4502 | hsa-miR-4526 | hsa-miR-4647 |
| hsa-miR-4503 | hsa-miR-4527 | hsa-miR-4648 |
| hsa-miR-4504 | hsa-miR-4528 | hsa-miR-4649 |
| hsa-miR-4505 | hsa-miR-4529 | hsa-miR-4650 |
| hsa-miR-4506 | hsa-miR-4530 | hsa-miR-4651 |
| hsa-miR-4507 | hsa-miR-4531 | hsa-miR-4652 |
| hsa-miR-4508 | hsa-miR-4532 | hsa-miR-4653 |
| hsa-miR-4509 | hsa-miR-4533 | hsa-miR-4654 |
| hsa-miR-450a-1 | hsa-miR-4534 | hsa-miR-4655 |
| hsa-miR-450a-2 | hsa-miR-4535 | hsa-miR-4656 |
| hsa-miR-450b | hsa-miR-4536 | hsa-miR-4657 |
| hsa-miR-4510 | hsa-miR-4537 | hsa-miR-4658 |
| hsa-miR-4511 | hsa-miR-4538 | hsa-miR-4659a |
| hsa-miR-4512 | hsa-miR-4539 | hsa-miR-4659b |
| hsa-miR-4513 | hsa-miR-454 | hsa-miR-466 |
| hsa-miR-4514 | hsa-miR-4540 | hsa-miR-4660 |
| hsa-miR-4515 | hsa-miR-455 | hsa-miR-4661 |
| hsa-miR-4516 | hsa-miR-4632 | hsa-miR-4662a |
| hsa-miR-4517 | hsa-miR-4633 | hsa-miR-4662b |
| hsa-miR-4518 | hsa-miR-4634 | hsa-miR-4663 |
| hsa-miR-4664 | hsa-miR-4695 | hsa-miR-4728 |
| hsa-miR-4665 | hsa-miR-4696 | hsa-miR-4729 |
| hsa-miR-4666a | hsa-miR-4697 | hsa-miR-4730 |
| hsa-miR-4666b | hsa-miR-4698 | hsa-miR-4731 |
| hsa-miR-4667 | hsa-miR-4699 | hsa-miR-4732 |
| hsa-miR-4668 | hsa-miR-4700 | hsa-miR-4733 |
| hsa-miR-4669 | hsa-miR-4701 | hsa-miR-4734 |
| hsa-miR-4670 | hsa-miR-4703 | hsa-miR-4735 |
| hsa-miR-4671 | hsa-miR-4704 | hsa-miR-4736 |
| hsa-miR-4672 | hsa-miR-4705 | hsa-miR-4737 |
| hsa-miR-4673 | hsa-miR-4706 | hsa-miR-4738 |
| hsa-miR-4674 | hsa-miR-4707 | hsa-miR-4739 |
| hsa-miR-4675 | hsa-miR-4708 | hsa-miR-4740 |
| hsa-miR-4676 | hsa-miR-4709 | hsa-miR-4741 |
| hsa-miR-4677 | hsa-miR-4710 | hsa-miR-4742 |
| hsa-miR-4678 | hsa-miR-4711 | hsa-miR-4743 |
| hsa-miR-4679 | hsa-miR-4712 | hsa-miR-4744 |
| hsa-miR-4680 | hsa-miR-4713 | hsa-miR-4745 |
| hsa-miR-4681 | hsa-miR-4714 | hsa-miR-4746 |
| hsa-miR-4682 | hsa-miR-4715 | hsa-miR-4747 |
| hsa-miR-4683 | hsa-miR-4716 | hsa-miR-4748 |
| hsa-miR-4684 | hsa-miR-4717 | hsa-miR-4749 |
| hsa-miR-4685 | hsa-miR-4718 | hsa-miR-4750 |
| hsa-miR-4686 | hsa-miR-4719 | hsa-miR-4751 |
| hsa-miR-4687 | hsa-miR-4720 | hsa-miR-4752 |
| hsa-miR-4688 | hsa-miR-4721 | hsa-miR-4753 |
| hsa-miR-4689 | hsa-miR-4722 | hsa-miR-4754 |
| hsa-miR-4690 | hsa-miR-4723 | hsa-miR-4755 |
| hsa-miR-4691 | hsa-miR-4724 | hsa-miR-4756 |
| hsa-miR-4692 | hsa-miR-4725 | hsa-miR-4757 |
| hsa-miR-4693 | hsa-miR-4726 | hsa-miR-4758 |
| hsa-miR-4694 | hsa-miR-4727 | hsa-miR-4759 |
| hsa-miR-4760 | hsa-miR-4792 | hsa-miR-499b |
| hsa-miR-4761 | hsa-miR-4793 | hsa-miR-5000 |
| hsa-miR-4762 | hsa-miR-4794 | hsa-miR-5001 |
| hsa-miR-4763 | hsa-miR-4795 | hsa-miR-5002 |
| hsa-miR-4764 | hsa-miR-4796 | hsa-miR-5003 |
| hsa-miR-4765 | hsa-miR-4797 | hsa-miR-5004 |
| hsa-miR-4766 | hsa-miR-4798 | hsa-miR-5006 |
| hsa-miR-4767 | hsa-miR-4799 | hsa-miR-5007 |
| hsa-miR-4768 | hsa-miR-4800 | hsa-miR-5008 |
| hsa-miR-4769 | hsa-miR-4801 | hsa-miR-5009 |
| hsa-miR-4770 | hsa-miR-4802 | hsa-miR-500a |
| hsa-miR-4771 | hsa-miR-4803 | hsa-miR-500b |
| hsa-miR-4772 | hsa-miR-4804 | hsa-miR-501 |
| hsa-miR-4773 | hsa-miR-483 | hsa-miR-5010 |
| hsa-miR-4774 | hsa-miR-484 | hsa-miR-5011 |
| hsa-miR-4775 | hsa-miR-485 | hsa-miR-502 |
| hsa-miR-4776 | hsa-miR-486 | hsa-miR-503 |
| hsa-miR-4777 | hsa-miR-487a | hsa-miR-504 |
| hsa-miR-4778 | hsa-miR-487b | hsa-miR-5047 |
| hsa-miR-4779 | hsa-miR-488 | hsa-miR-505 |
| hsa-miR-4780 | hsa-miR-489 | hsa-miR-506 |
| hsa-miR-4781 | hsa-miR-490 | hsa-miR-507 |
| hsa-miR-4782 | hsa-miR-491 | hsa-miR-508 |
| hsa-miR-4783 | hsa-miR-492 | hsa-miR-5087 |
| hsa-miR-4784 | hsa-miR-493 | hsa-miR-5088 |
| hsa-miR-4785 | hsa-miR-494 | hsa-miR-5089 |
| hsa-miR-4786 | hsa-miR-495 | hsa-miR-509-1 |
| hsa-miR-4787 | hsa-miR-496 | hsa-miR-509-3 |
| hsa-miR-4788 | hsa-miR-497 | hsa-miR-5090 |
| hsa-miR-4789 | hsa-miR-498 | hsa-miR-5091 |
| hsa-miR-4790 | hsa-miR-4999 | hsa-miR-5092 |
| hsa-miR-4791 | hsa-miR-499a | hsa-miR-5093 |
| hsa-miR-5094 | hsa-miR-5194 | hsa-miR-545 |
| hsa-miR-5095 | hsa-miR-5195 | hsa-miR-548a |
| hsa-miR-5096 | hsa-miR-5196 | hsa-miR-548aa |
| hsa-miR-510 | hsa-miR-5197 | hsa-miR-548ab |
| hsa-miR-5100 | hsa-miR-519a | hsa-miR-548ac |
| hsa-miR-511 | hsa-miR-519b | hsa-miR-548ad |
| hsa-miR-512 | hsa-miR-519c | hsa-miR-548ae |
| hsa-miR-513a | hsa-miR-519d | hsa-miR-548ag |
| hsa-miR-513b | hsa-miR-519e | hsa-miR-548ah |
| hsa-miR-513c | hsa-miR-520a | hsa-miR-548ai |
| hsa-miR-514a | hsa-miR-520b | hsa-miR-548aj |
| hsa-miR-514b | hsa-miR-520c | hsa-miR-548ak |
| hsa-miR-515 | hsa-miR-520d | hsa-miR-548al |
| hsa-miR-516a | hsa-miR-520e | hsa-miR-548am |
| hsa-miR-516b | hsa-miR-520f | hsa-miR-548an |
| hsa-miR-517a | hsa-miR-520g | hsa-miR-548ao |
| hsa-miR-517b | hsa-miR-520h | hsa-miR-548ap |
| hsa-miR-517c | hsa-miR-521 | hsa-miR-548aq |
| hsa-miR-5186 | hsa-miR-522 | hsa-miR-548ar |
| hsa-miR-5187 | hsa-miR-523 | hsa-miR-548as |
| hsa-miR-5188 | hsa-miR-524 | hsa-miR-548at |
| hsa-miR-5189 | hsa-miR-525 | hsa-miR-548au |
| hsa-miR-518a | hsa-miR-526a | hsa-miR-548av |
| hsa-miR-518b | hsa-miR-526b | hsa-miR-548aw |
| hsa-miR-518c | hsa-miR-527 | hsa-miR-548ax |
| hsa-miR-518d | hsa-miR-532 | hsa-miR-548ay |
| hsa-miR-518e | hsa-miR-539 | hsa-miR-548az |
| hsa-miR-518f | hsa-miR-541 | hsa-miR-548b |
| hsa-miR-5190 | hsa-miR-542 | hsa-miR-548ba |
| hsa-miR-5191 | hsa-miR-543 | hsa-miR-548bb |
| hsa-miR-5192 | hsa-miR-544a | hsa-miR-548c |
| hsa-miR-5193 | hsa-miR-544b | hsa-miR-548d |
| hsa-miR-548e | hsa-miR-557 | hsa-miR-5687 |
| hsa-miR-548f | hsa-miR-5571 | hsa-miR-5688 |
| hsa-miR-548g | hsa-miR-5572 | hsa-miR-5689 |
| hsa-miR-548h | hsa-miR-5579 | hsa-miR-569 |
| hsa-miR-548i | hsa-miR-558 | hsa-miR-5690 |
| hsa-miR-548j | hsa-miR-5580 | hsa-miR-5691 |
| hsa-miR-548k | hsa-miR-5581 | hsa-miR-5692a |
| hsa-miR-548l | hsa-miR-5582 | hsa-miR-5692b |
| hsa-miR-548m | hsa-miR-5583 | hsa-miR-5692c |
| hsa-miR-548n | hsa-miR-5584 | hsa-miR-5693 |
| hsa-miR-548o | hsa-miR-5585 | hsa-miR-5694 |
| hsa-miR-548p | hsa-miR-5586 | hsa-miR-5695 |
| hsa-miR-548q | hsa-miR-5587 | hsa-miR-5696 |
| hsa-miR-548s | hsa-miR-5588 | hsa-miR-5697 |
| hsa-miR-548t | hsa-miR-5589 | hsa-miR-5698 |
| hsa-miR-548u | hsa-miR-559 | hsa-miR-5699 |
| hsa-miR-548v | hsa-miR-5590 | hsa-miR-570 |
| hsa-miR-548w | hsa-miR-5591 | hsa-miR-5700 |
| hsa-miR-548x | hsa-miR-561 | hsa-miR-5701 |
| hsa-miR-548y | hsa-miR-562 | hsa-miR-5702 |
| hsa-miR-548z | hsa-miR-563 | hsa-miR-5703 |
| hsa-miR-549a | hsa-miR-564 | hsa-miR-5704 |
| hsa-miR-550a-1 | hsa-miR-566 | hsa-miR-5705 |
| hsa-miR-550a-3 | hsa-miR-567 | hsa-miR-5706 |
| hsa-miR-550b | hsa-miR-568 | hsa-miR-5707 |
| hsa-miR-551a | hsa-miR-5680 | hsa-miR-5708 |
| hsa-miR-551b | hsa-miR-5681a | hsa-miR-571 |
| hsa-miR-552 | hsa-miR-5681b | hsa-miR-572 |
| hsa-miR-553 | hsa-miR-5682 | hsa-miR-573 |
| hsa-miR-554 | hsa-miR-5683 | hsa-miR-5739 |
| hsa-miR-555 | hsa-miR-5684 | hsa-miR-574 |
| hsa-miR-556 | hsa-miR-5685 | hsa-miR-575 |
| hsa-miR-576 | hsa-miR-6069 | hsa-miR-6128 |
| hsa-miR-577 | hsa-miR-607 | hsa-miR-6129 |
| hsa-miR-578 | hsa-miR-6070 | hsa-miR-613 |
| hsa-miR-5787 | hsa-miR-6071 | hsa-miR-6130 |
| hsa-miR-579 | hsa-miR-6072 | hsa-miR-6131 |
| hsa-miR-580 | hsa-miR-6073 | hsa-miR-6132 |
| hsa-miR-581 | hsa-miR-6074 | hsa-miR-6133 |
| hsa-miR-582 | hsa-miR-6075 | hsa-miR-6134 |
| hsa-miR-583 | hsa-miR-6076 | hsa-miR-614 |
| hsa-miR-584 | hsa-miR-6077 | hsa-miR-615 |
| hsa-miR-585 | hsa-miR-6078 | hsa-miR-616 |
| hsa-miR-586 | hsa-miR-6079 | hsa-miR-6165 |
| hsa-miR-587 | hsa-miR-608 | hsa-miR-617 |
| hsa-miR-588 | hsa-miR-6080 | hsa-miR-618 |
| hsa-miR-589 | hsa-miR-6081 | hsa-miR-619 |
| hsa-miR-590 | hsa-miR-6082 | hsa-miR-620 |
| hsa-miR-591 | hsa-miR-6083 | hsa-miR-621 |
| hsa-miR-592 | hsa-miR-6084 | hsa-miR-622 |
| hsa-miR-593 | hsa-miR-6085 | hsa-miR-623 |
| hsa-miR-595 | hsa-miR-6086 | hsa-miR-624 |
| hsa-miR-596 | hsa-miR-6087 | hsa-miR-625 |
| hsa-miR-597 | hsa-miR-6088 | hsa-miR-626 |
| hsa-miR-598 | hsa-miR-6089 | hsa-miR-627 |
| hsa-miR-599 | hsa-miR-609 | hsa-miR-628 |
| hsa-miR-600 | hsa-miR-6090 | hsa-miR-629 |
| hsa-miR-601 | hsa-miR-610 | hsa-miR-630 |
| hsa-miR-602 | hsa-miR-611 | hsa-miR-631 |
| hsa-miR-603 | hsa-miR-612 | hsa-miR-632 |
| hsa-miR-604 | hsa-miR-6124 | hsa-miR-633 |
| hsa-miR-605 | hsa-miR-6125 | hsa-miR-634 |
| hsa-miR-606 | hsa-miR-6126 | hsa-miR-635 |
| hsa-miR-6068 | hsa-miR-6127 | hsa-miR-636 |
| hsa-miR-637 | hsa-miR-6514 | hsa-miR-6724 |
| hsa-miR-638 | hsa-miR-6515 | hsa-miR-6726 |
| hsa-miR-639 | hsa-miR-6516 | hsa-miR-6727 |
| hsa-miR-640 | hsa-miR-652 | hsa-miR-6728 |
| hsa-miR-641 | hsa-miR-653 | hsa-miR-6729 |
| hsa-miR-642a | hsa-miR-654 | hsa-miR-6730 |
| hsa-miR-642b | hsa-miR-655 | hsa-miR-6731 |
| hsa-miR-643 | hsa-miR-656 | hsa-miR-6732 |
| hsa-miR-644a | hsa-miR-657 | hsa-miR-6733 |
| hsa-miR-645 | hsa-miR-658 | hsa-miR-6734 |
| hsa-miR-646 | hsa-miR-659 | hsa-miR-6735 |
| hsa-miR-647 | hsa-miR-660 | hsa-miR-6736 |
| hsa-miR-648 | hsa-miR-661 | hsa-miR-6737 |
| hsa-miR-649 | hsa-miR-662 | hsa-miR-6738 |
| hsa-miR-6499 | hsa-miR-663a | hsa-miR-6739 |
| hsa-miR-650 | hsa-miR-663b | hsa-miR-6740 |
| hsa-miR-6500 | hsa-miR-664a | hsa-miR-6741 |
| hsa-miR-6501 | hsa-miR-664b | hsa-miR-6742 |
| hsa-miR-6502 | hsa-miR-665 | hsa-miR-6743 |
| hsa-miR-6503 | hsa-miR-668 | hsa-miR-6744 |
| hsa-miR-6504 | hsa-miR-670 | hsa-miR-6745 |
| hsa-miR-6505 | hsa-miR-671 | hsa-miR-6746 |
| hsa-miR-6506 | hsa-miR-6715a | hsa-miR-6747 |
| hsa-miR-6507 | hsa-miR-6715b | hsa-miR-6748 |
| hsa-miR-6508 | hsa-miR-6716 | hsa-miR-6749 |
| hsa-miR-6509 | hsa-miR-6717 | hsa-miR-675 |
| hsa-miR-651 | hsa-miR-6718 | hsa-miR-6750 |
| hsa-miR-6510 | hsa-miR-6719 | hsa-miR-6751 |
| hsa-miR-6511a | hsa-miR-6720 | hsa-miR-6752 |
| hsa-miR-6511b | hsa-miR-6721 | hsa-miR-6753 |
| hsa-miR-6512 | hsa-miR-6722 | hsa-miR-6754 |
| hsa-miR-6513 | hsa-miR-6723 | hsa-miR-6755 |
| hsa-miR-6756 | hsa-miR-6785 | hsa-miR-6817 |
| hsa-miR-6757 | hsa-miR-6786 | hsa-miR-6818 |
| hsa-miR-6758 | hsa-miR-6787 | hsa-miR-6819 |
| hsa-miR-6759 | hsa-miR-6788 | hsa-miR-6820 |
| hsa-miR-676 | hsa-miR-6789 | hsa-miR-6821 |
| hsa-miR-6760 | hsa-miR-6790 | hsa-miR-6822 |
| hsa-miR-6761 | hsa-miR-6791 | hsa-miR-6823 |
| hsa-miR-6762 | hsa-miR-6792 | hsa-miR-6824 |
| hsa-miR-6763 | hsa-miR-6793 | hsa-miR-6825 |
| hsa-miR-6764 | hsa-miR-6794 | hsa-miR-6826 |
| hsa-miR-6765 | hsa-miR-6795 | hsa-miR-6827 |
| hsa-miR-6766 | hsa-miR-6796 | hsa-miR-6828 |
| hsa-miR-6767 | hsa-miR-6797 | hsa-miR-6829 |
| hsa-miR-6768 | hsa-miR-6798 | hsa-miR-6830 |
| hsa-miR-6769a | hsa-miR-6799 | hsa-miR-6831 |
| hsa-miR-6769b | hsa-miR-6800 | hsa-miR-6832 |
| hsa-miR-6770 | hsa-miR-6801 | hsa-miR-6833 |
| hsa-miR-6771 | hsa-miR-6802 | hsa-miR-6834 |
| hsa-miR-6772 | hsa-miR-6803 | hsa-miR-6835 |
| hsa-miR-6773 | hsa-miR-6804 | hsa-miR-6836 |
| hsa-miR-6774 | hsa-miR-6805 | hsa-miR-6837 |
| hsa-miR-6775 | hsa-miR-6806 | hsa-miR-6838 |
| hsa-miR-6776 | hsa-miR-6807 | hsa-miR-6839 |
| hsa-miR-6777 | hsa-miR-6808 | hsa-miR-6840 |
| hsa-miR-6778 | hsa-miR-6809 | hsa-miR-6841 |
| hsa-miR-6779 | hsa-miR-6810 | hsa-miR-6842 |
| hsa-miR-6780a | hsa-miR-6811 | hsa-miR-6843 |
| hsa-miR-6780b | hsa-miR-6812 | hsa-miR-6844 |
| hsa-miR-6781 | hsa-miR-6813 | hsa-miR-6845 |
| hsa-miR-6782 | hsa-miR-6814 | hsa-miR-6846 |
| hsa-miR-6783 | hsa-miR-6815 | hsa-miR-6847 |
| hsa-miR-6784 | hsa-miR-6816 | hsa-miR-6848 |
| hsa-miR-6849 | hsa-miR-6881 | hsa-miR-7154 |
| hsa-miR-6850 | hsa-miR-6882 | hsa-miR-7155 |
| hsa-miR-6851 | hsa-miR-6883 | hsa-miR-7156 |
| hsa-miR-6852 | hsa-miR-6884 | hsa-miR-7157 |
| hsa-miR-6853 | hsa-miR-6885 | hsa-miR-7158 |
| hsa-miR-6854 | hsa-miR-6886 | hsa-miR-7159 |
| hsa-miR-6855 | hsa-miR-6887 | hsa-miR-7160 |
| hsa-miR-6856 | hsa-miR-6888 | hsa-miR-7161 |
| hsa-miR-6857 | hsa-miR-6889 | hsa-miR-7162 |
| hsa-miR-6858 | hsa-miR-6890 | hsa-miR-718 |
| hsa-miR-6859 | hsa-miR-6891 | hsa-miR-744 |
| hsa-miR-6860 | hsa-miR-6892 | hsa-miR-7515 |
| hsa-miR-6861 | hsa-miR-6893 | hsa-miR-758 |
| hsa-miR-6862 | hsa-miR-6894 | hsa-miR-759 |
| hsa-miR-6863 | hsa-miR-6895 | hsa-miR-760 |
| hsa-miR-6864 | hsa-miR-7-1 | hsa-miR-761 |
| hsa-miR-6865 | hsa-miR-7-2 | hsa-miR-762 |
| hsa-miR-6866 | hsa-miR-708 | hsa-miR-764 |
| hsa-miR-6867 | hsa-miR-7106 | hsa-miR-7641 |
| hsa-miR-6868 | hsa-miR-7107 | hsa-miR-765 |
| hsa-miR-6869 | hsa-miR-7108 | hsa-miR-766 |
| hsa-miR-6870 | hsa-miR-7109 | hsa-miR-767 |
| hsa-miR-6871 | hsa-miR-711 | hsa-miR-769 |
| hsa-miR-6872 | hsa-miR-7110 | hsa-miR-770 |
| hsa-miR-6873 | hsa-miR-7111 | hsa-miR-7702 |
| hsa-miR-6874 | hsa-miR-7112 | hsa-miR-7703 |
| hsa-miR-6875 | hsa-miR-7113 | hsa-miR-7704 |
| hsa-miR-6876 | hsa-miR-7114 | hsa-miR-7705 |
| hsa-miR-6877 | hsa-miR-7150 | hsa-miR-7706 |
| hsa-miR-6878 | hsa-miR-7151 | hsa-miR-7843 |
| hsa-miR-6879 | hsa-miR-7152 | hsa-miR-7844 |
| hsa-miR-6880 | hsa-miR-7153 | hsa-miR-7845 |
| hsa-miR-7846 | hsa-miR-8066 | hsa-miR-888 |
| hsa-miR-7847 | hsa-miR-8067 | hsa-miR-889 |
| hsa-miR-7848 | hsa-miR-8068 | hsa-miR-890 |
| hsa-miR-7849 | hsa-miR-8069 | hsa-miR-891a |
| hsa-miR-7850 | hsa-miR-8070 | hsa-miR-891b |
| hsa-miR-7851 | hsa-miR-8071 | hsa-miR-892a |
| hsa-miR-7852 | hsa-miR-8072 | hsa-miR-892b |
| hsa-miR-7853 | hsa-miR-8073 | hsa-miR-892c |
| hsa-miR-7854 | hsa-miR-8074 | hsa-miR-9 |
| hsa-miR-7855 | hsa-miR-8075 | hsa-miR-920 |
| hsa-miR-7856 | hsa-miR-8076 | hsa-miR-921 |
| hsa-miR-7973 | hsa-miR-8077 | hsa-miR-922 |
| hsa-miR-7974 | hsa-miR-8078 | hsa-miR-924 |
| hsa-miR-7975 | hsa-miR-8079 | hsa-miR-92a-1 |
| hsa-miR-7976 | hsa-miR-8080 | hsa-miR-92a-2 |
| hsa-miR-7977 | hsa-miR-8081 | hsa-miR-92b |
| hsa-miR-7978 | hsa-miR-8082 | hsa-miR-93 |
| hsa-miR-802 | hsa-miR-8083 | hsa-miR-933 |
| hsa-miR-8052 | hsa-miR-8084 | hsa-miR-934 |
| hsa-miR-8053 | hsa-miR-8085 | hsa-miR-935 |
| hsa-miR-8054 | hsa-miR-8086 | hsa-miR-936 |
| hsa-miR-8055 | hsa-miR-8087 | hsa-miR-937 |
| hsa-miR-8056 | hsa-miR-8088 | hsa-miR-938 |
| hsa-miR-8057 | hsa-miR-8089 | hsa-miR-939 |
| hsa-miR-8058 | hsa-miR-8485 | hsa-miR-940 |
| hsa-miR-8059 | hsa-miR-873 | hsa-miR-941 |
| hsa-miR-8060 | hsa-miR-874 | hsa-miR-942 |
| hsa-miR-8061 | hsa-miR-875 | hsa-miR-943 |
| hsa-miR-8062 | hsa-miR-876 | hsa-miR-944 |
| hsa-miR-8063 | hsa-miR-877 | hsa-miR-95 |
| hsa-miR-8064 | hsa-miR-885 | hsa-miR-9500 |
| hsa-miR-8065 | hsa-miR-887 | hsa-miR-96 |
| hsa-miR-98 | hsa-miR-99a | hsa-miR-99b |

### 1-2: Cell culture and miRNA transfection

In order to discover miRNAs that induces melanin production, human melanoma M21 (Korean cell line bank, KR), which is a human-derived skin cancer cell line, was used. The M21 cells were cultured at 37°C/5% CO₂ in DMEM (Hyclone, US) supplemented with 10% fetal bovine serum (FBS, Hyclone, US) and 1% penicillin-streptomycin (Hyclone, US). M21 cells were dispensed at 4×10⁴ cells/well in a 12-well plate (Falcon, US), and the next day, were transfected with miRNA at 40 nM using Lipofectamine RNAiMAX (Invitrogen, US) according to the manufacturer's protocol.

### 1-3: Screening from 1728 miRNAs by measuring amount of melanin production

M21 cells were transfected three times with 1728 miRNAs by the method of Example 1-2. After culture for 72 hours, all cells were collected and allowed to react in 1 M NaOH (Bioneer, KR) for 2 hours at 37°C according to the melanin extraction protocol, after which absorbance was measured at a wavelength of 405 mm to compare color change and the amount of melanin produced. After culture for 72 hours, all cells were collected and allowed to react in 1 M NaOH (Bioneer, KR) for 2 hours at 37°C according to the melanin extraction protocol, after which absorbance was measured at a wavelength of 405 mm and thus the amount of melanin produced was analyzed. In order to evaluate reproducibility, the top 9 miRNAs were used to treat the M21 cells by the method of Example 1-2. After culture for 72 hours, all cells were collected and allowed to react in 1 M NaOH (Bioneer, KR) for 2 hours at 37°C according to the melanin extraction protocol, after which absorbance was measured at a wavelength of 405 mm and thus the amount of melanin produced was analyzed.

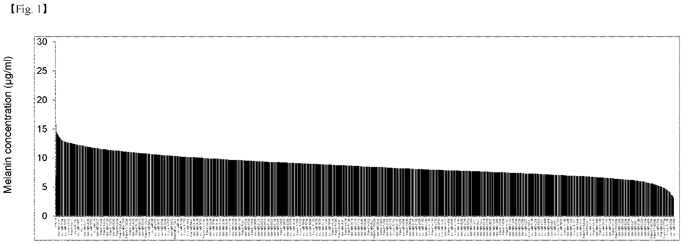

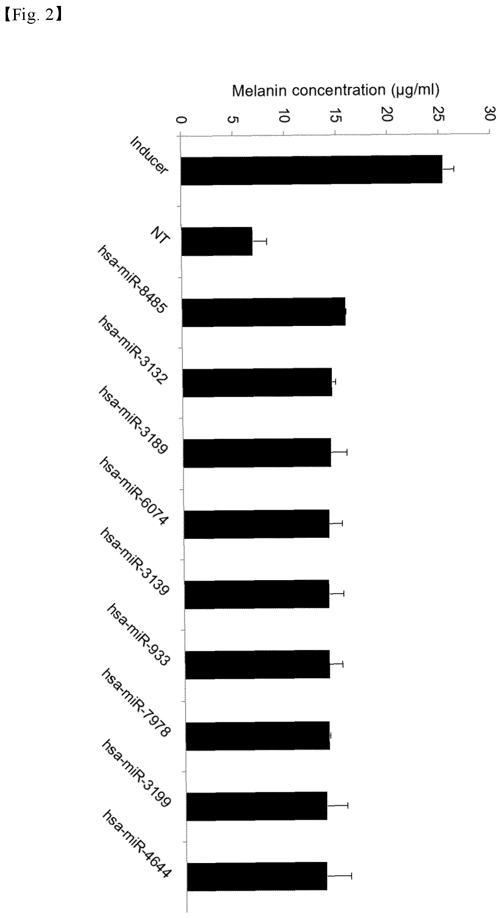

Thereby, in the M21 cells treated with a total of 1728 miRNAs, 9 miRNAs showing melanin produced in an amount of 13.0 pg/ml or more (1.88 times or more compared to the negative control) and the amount of melanin produced similar to the positive control IBMX (Sigma, US)/CuSO₄ (Sigma, US) treatment group were selected (FIG. 1). In repeated experiments for reproducibility evaluation, color change and melanogenesis were confirmed in the M21 cell line by the selected top 9 miRNAs compared to the negative control group (FIG. 2, Table 3).

**[Table 3] Top 9 miRNA sequences for melanogenesis**

| | | | |
|---|---|---|---|
| SEQ ID NO: 1 | hsa-miR-8485 | Strand 1 | ACGUGUGUGUGUGUGUGUGUU |
| SEQ ID NO: 2 | hsa-miR-8485 | Strand 2 | CACACACACACACACACGUAU |
| SEQ ID NO: 3 | hsa-miR-3132 | Strand 1 | CUCUGAGCUCCUUCUCUACCCAUU |
| SEQ ID NO: 4 | hsa-miR-3132 | Strand 2 | UGGGUAGAGAAGGAGCUCAGAGGA |
| SEQ ID NO: 5 | hsa-miR-3189 | Strand 1 | UGCCCCAUCUGUGCCCUGGGUAGGA |
| SEQ ID NO: 6 | hsa-miR-3189 | Strand 2 | CCCUUGGGUCUGAUGGGGUAG |
| SEQ ID NO: 7 | hsa-miR-6074 | Strand 1 | ACCUAGCCUCUGAAUAUCUU |
| SEQ ID NO: 8 | hsa-miR-6074 | Strand 2 | GAUAUUCAGAGGCUAGGUGG |
| SEQ ID NO: 9 | hsa-miR-3139 | Strand 1 | CAGGCAUCUGUUGAGCUCCUAUU |
| SEQ ID NO: 10 | hsa-miR-3139 | Strand 2 | UAGGAGCUCAACAGAUGCCUGUU |
| SEQ ID NO: 11 | hsa-miR-933 | Strand 1 | GAGAGGUCUCCCUGCGCACAUU |
| SEQ ID NO: 12 | hsa-miR-933 | Strand 2 | UGUGCGCAGGGAGACCUCUCCC |
| SEQ ID NO: 13 | hsa-miR-7978 | Strand 1 | AGCAACGCUAUACACCAGAUU |
| SEQ ID NO: 14 | hsa-miR-7978 | Strand 2 | UCUGGUGUAUAGCGUUGCUCA |
| SEQ ID NO: 15 | hsa-miR-3199 | Strand 1 | CUUUCUCCUAAGGCAGUCCCUUU |
| SEQ ID NO: 16 | hsa-miR-3199 | Strand 2 | AGGGACUGCCUUAGGAGAAAGUU |
| SEQ ID NO: 17 | hsa-miR-4644 | Strand 1 | UCUGUCUCUUUUCUCUCUCCAUU |
| SEQ ID NO: 18 | hsa-miR-4644 | Strand 2 | UGGAGAGAGAAAAGAGACAGAAG |

### Example 2: Final melanin-inducing miRNA selection through reproducibility evaluation of 9 selected miRNAs and analysis of melanogenesis mechanism

### 2-1: Cell culture and miRNA transfection

SK-MEL-28 (Korean cell line bank, KR), which is a human-derived skin cancer cell line, was used to evaluate reproducibility of the top 9 miRNAs for melanogenesis. The SK-MEL-28 cells were cultured at 37°C/5% CO₂ in MEM (Hyclone, US) supplemented with 10% FBS (Hyclone, US) and 1% penicillin-streptomycin (Hyclone, US). SK-MEL-28 cells were dispensed at 4×10⁴ cells/well in a 12-well plate (Falcon, US), and the next day, were transfected with miRNA at 40 nM using Lipofectamine RNAiMAX (Invitrogen, US) according to the manufacturer's protocol.

### 2-2: Evaluation of reproducibility of top 9 miRNAs through analysis of amount of melanin produced

In order to evaluate reproducibility of the selected top 9 miRNAs, color change and the amount of melanin produced were measured in SK-MEL-28 cells. SK-MEL-28 cells were transfected three times with each of miR-8485, miR-7978, miR-6074, miR-3132, miR-4644, miR-3139, miR-3189, miR-3199, and miR-933 by the method of Example 2-1. After culture for 72 hours, all cells were collected and color changes were observed. In order to measure the amount of melanin produced, reaction was carried out in 1 M NaOH (Bioneer, KR) for 2 hours at 37°C according to the melanin extraction protocol, after which absorbance was measured at a wavelength of 405 mm and thus the amount of melanin produced was analyzed.

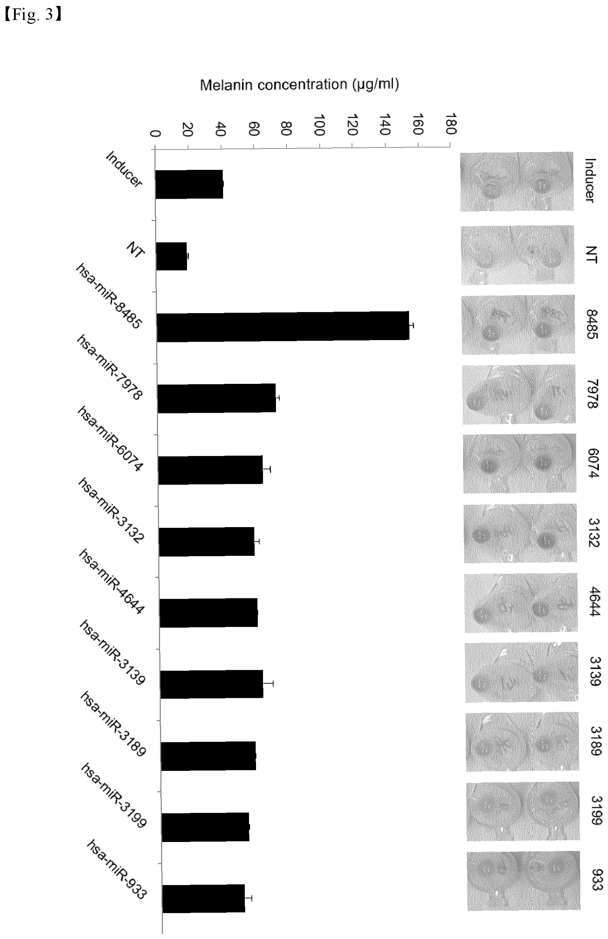

Thereby, in the group treated with miR-8485, miR-7978, miR-6074, miR-3132, miR-4644, miR-3139, miR-3189, miR-3199, or miR-933, a distinct color change and an increase in the amount of melanin produced were observed compared to the negative control group. A significant color change and an increase in the amount of melanin produced were confirmed when compared to the positive control melanin inducer (FIG. 3).

### 2-3: Analysis of melanogenesis signaling by RT-qPCR in SK-MEL-28 cells

In order to analyze the mechanism of action of the selected top 9 miRNAs for promoting melanogenesis, RT-qPCR was performed for gene analysis of MITF, TYR, TYRP1, and TYRP2, which are key factors in the melanogenesis. The SK-MEL-28 cells were dispensed at 4×10⁴ cells/well in a 12-well plate (Falcon, US) and then cultured at 37°C/5% CO₂. The next day, by the method of Example 2-1, SK-MEL-28 cells were transfectedthree times with each of miR-8485, miR-7978, miR-6074, miR-3132, miR-4644, miR-3139, miR-3189, miR-3199, and miR-933. After culture for 96 hours, total RNA was extracted from the cell lysate using a Universal RNA extraction kit (Bioneer, KR), and using this RNA as a template, mRNA expression levels of the genes (Human qPCR panel kit, Bioneer, KR) and RPL13A (Human reference qPCR primer set, Bioneer, KR) were analyzed by qRT-PCR using *AccuPower* GreenStar^{™} RT-qPCR Master Mix (Bioneer, KR) according to the manufacturer's protocol. The Ct values of two genes derived after qPCR array were subjected to relative quantitative analysis through a 2(-Delta Delta C(T)) method [Livak KJ, Schmittgen TD. 2001. Methods. Dec;25(4):4 02-8], so that the relative amount (fold change) of each gene mRNA in the experimental group compared to the control group was calculated. Primer sequences for individual genes are shown below (Table 4).

**[Table 4] Human MITF, TYR, TYRP1/2, RPL13A (internal control) primer sequences**

| | | |
|---|---|---|
| SEQ ID NO: 19 | hMITF-forward | 5'- CGTCTCTCACTGGATTGGTG -3' |
| SEQ ID NO: 20 | hMITF-reverse | 5'- CTTATAAAATCCCTGCTGCCGT -3' |
| SEQ ID NO: 21 | hTYR-forward | 5'- GGATAGCGGATGCCTCTCAA -3' |
| SEQ ID NO: 22 | hTYR-reverse | 5'- GGAGCCACTGCTCAAAAATAC -3' |
| SEQ ID NO: 23 | hTYRP1-forward | 5'- CCACAGCCCTCAGTATCCC -3' |
| SEQ ID NO: 24 | hTYRP1-reverse | 5'- CAGCTCCTCTCCAGCCAG -3' |
| SEQ ID NO: 25 | hTYRP2-forward | 5'- TCGGATGTACAACATGGTTCC -3' |
| SEQ ID NO: 26 | hTYRP2-reverse | 5'- CCAACCTGGAGTTTCTTCAAC -3' |
| SEQ ID NO: 27 | hRPL13A-forward | 5'- CCAGCAATCAAGTTTGCCTA -3' |
| SEQ ID NO: 28 | hRPL13A-reverse | 5'- GTGGTGGTGGTGGTAATTCA -3' |

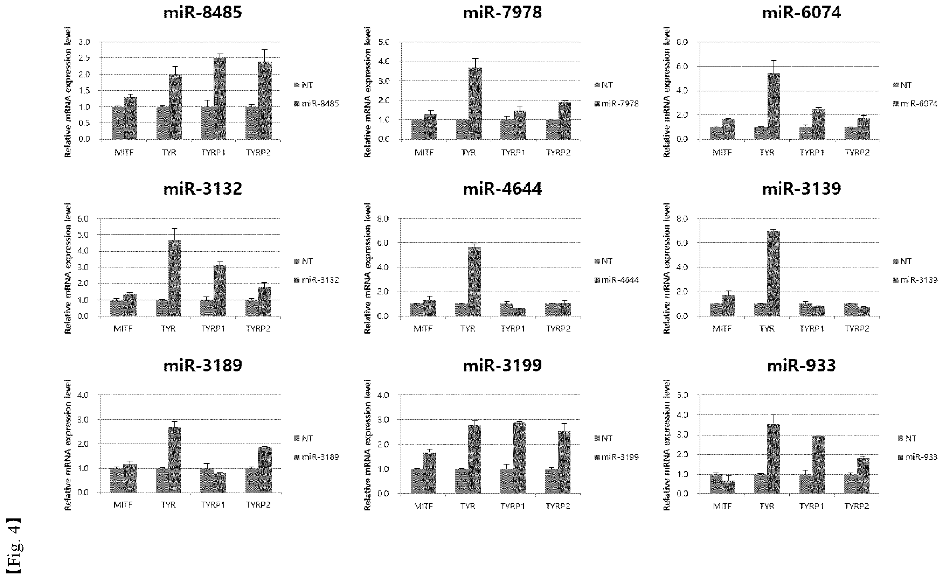

Thereby, it was confirmed that the gene expression of TYR, TYRP1, and TYRP2, which are enzymes directly involved in melanin synthesis in melanosomes, was increased by 9 miRNAs (FIG. 4). In case of MITF, which is a transcription factor, there was a weak increase or no change in gene expression. This is deemed to be due to the presence of a MITF regulation mechanism in which not only gene expression but also protein stability and activity.

### 2-4: Analysis of melanogenesis signaling by RT-qPCR in human melanocytes

The gene expression of MITF, TYR, TYRP1, and TYRP2 by 9 miRNAs was analyzed using human epidermal melanocytes obtained from human wild-type skin tissue. Human epidermal melanocytes (Invitrogen, US) were dispensed at 1×10⁵ cells/well into a 12-well plate (Falcon, US), followed by culture at 37°C/5% CO₂ in mammalian cell culture/primary cell culture (Gibco, US) containing human melanocyte growth supplement-2 (Gibco, US). The next day, the cells were treated with each of miR-8485, miR-7978, miR-6074, miR-3132, miR-4644, miR-3139, miR-3189, miR-3199, and miR-933 at a concentration of 5 µM. After culture for 96 hours, total RNA was extracted from the cell lysate using a Universal RNA extraction kit (Bioneer, KR), and using this RNA as a template, mRNA expression levels of the genes (Human qPCR panel kit, Bioneer, KR) and RPL13A (Human reference qPCR primer set, Bioneer, KR) were analyzed by qRT-PCR using *AccuPower* GreenStar^{™} RT-qPCR Master Mix (Bioneer, KR) according to the manufacturer's protocol.

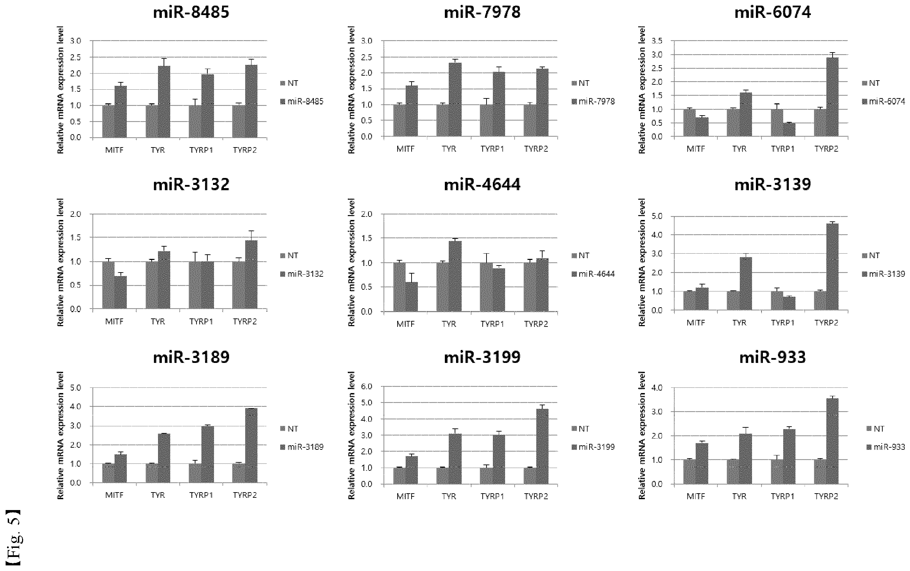

Thereby, in human wild-type melanocytes, unlike the results of M21 melanoma cells, the gene expression of MITF by miR-8485, miR-7978, miR-3139, miR-3189, miR-3199, or miR-933 was increased about 1.2 to 1.7 times compared to the negative control group. Also, the expression of TYR was increased about 1.2 to 3.1 times by 9 miRNAs. For TYRP1 and TYRP2, differences in expression patterns were observed by each of the 9 miRNA. For miR-8485, miR-7978, miR-3189, miR-3199, and miR-933, both TYRP1 and TYRP2 expressions tended to increase, and for miR-6074, miR-3139, and miR-4644, only TYRP2 expression was increased (FIG. 5).

### 2-5: Analysis of melanogenesis signaling by western blotting in SK-MEL-28 cells

Western blotting was performed to analyze expression of MITF and TYR proteins, which are important factors in the melanogenesis process. The SK-MEL-28 cells were dispensed at 1×10⁵ cells/well in a 6-well plate (Falcon, US) and then cultured at 37°C/5% CO₂. The next day, the SK-MEL-28 cells were transfected with each of miR-3139, miR-3189, miR-3199, and miR-8485 by the method of Example 2-1. After culture for 96 hours, analysis was performed according to a Western blotting protocol. MITF (Santa Cruz Biotechnology, Inc, US) and tyrosinase (Santa Cruz Biotechnology, Inc, US) were used as primary antibodies, and HRP-linked-anti-mouse IgG (Cell Signaling Technology, US) and HRP-linked-anti-rabbit IgG (Cell Signaling Technology, US) were used as secondary antibodies.

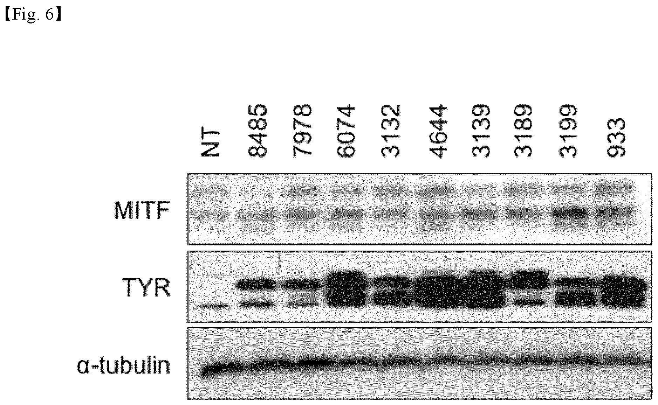

Thereby, it was confirmed that the expression of MITF and TYR proteins was increased by 9 miRNAs, like the results of RT-qPCR above (FIG. 6). Based on the results of cell color change, the amount of melanin synthesized, and the expression of major genes and proteins in the melanogenesis by treatment with the selected top 9 miRNAs, 4 types of miR-3139, miR-3189, miR-3199, and miR-8485 with the highest amount of melanin synthesized and clear gene and protein expression of MITF, TYR, and TYRP1/2 were finally selected (Table 5).

**[Table 5] Finally selected 4 miRNA sequences**

| | | | |
|---|---|---|---|
| SEQ ID NO:9 | hsa-miR-3139 | Strand 1 | CAGGCAUCUGUUGAGCUCCUAUU |
| SEQ ID NO:10 | hsa-miR-3139 | Strand 2 | UAGGAGCUCAACAGAUGCCUGUU |
| SEQ ID NO:5 | hsa-miR-3189 | Strand 1 | UGCCCCAUCUGUGCCCUGGGUAGGA |
| SEQ ID NO:6 | hsa-miR-3189 | Strand 2 | CCCUUGGGUCUGAUGGGGUAG |
| SEQ ID NO:15 | hsa-miR-3199 | Strand 1 | CUUUCUCCUAAGGCAGUCCCUUU |
| SEQ ID NO:16 | hsa-miR-3199 | Strand 2 | AGGGACUGCCUUAGGAGAAAGUU |
| SEQ ID NO:1 | hsa-miR-8485 | Strand 1 | ACGUGUGUGUGUGUGUGUGUU |
| SEQ ID NO:2 | hsa-miR-8485 | Strand 2 | CACACACACACACACAC GUAU |

### Example 3: Evaluation of melanogenic function of finally selected 4 miRNAs on plucked human gray hair

Melanogenic function was evaluated for plucked human gray hair using the finally selected 4 miRNAs. Hair was collected by pulling white hair from the tip thereof on the day of the experiment, and the hair was cut by about 1 to 2 cm from the hair root and cultured in 200 µL of DMEM/F12 medium (Gibco, US) supplemented with 10% FBS (Hyclone, US) and 1% penicillin-streptomycin (Hyclone, US) in a 24-well plate (Falcon, US). The plucked gray hairs were treated with each of 4 miRNAs at a concentration of 5 µM for 35 days at 2-day intervals, and hair follicle-derived primary cell proliferation, hair color change, and appearance of gray hair were observed.

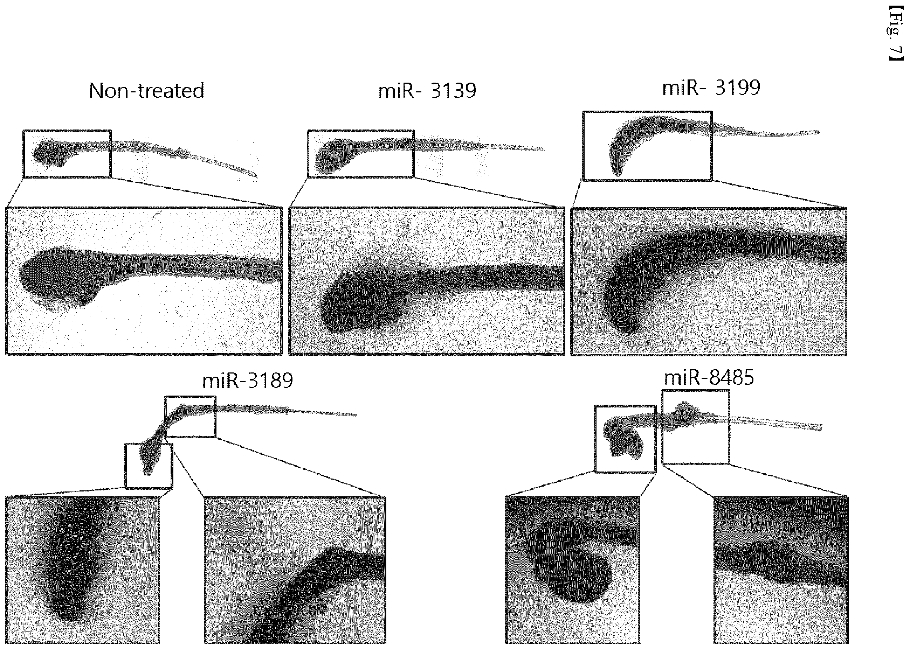

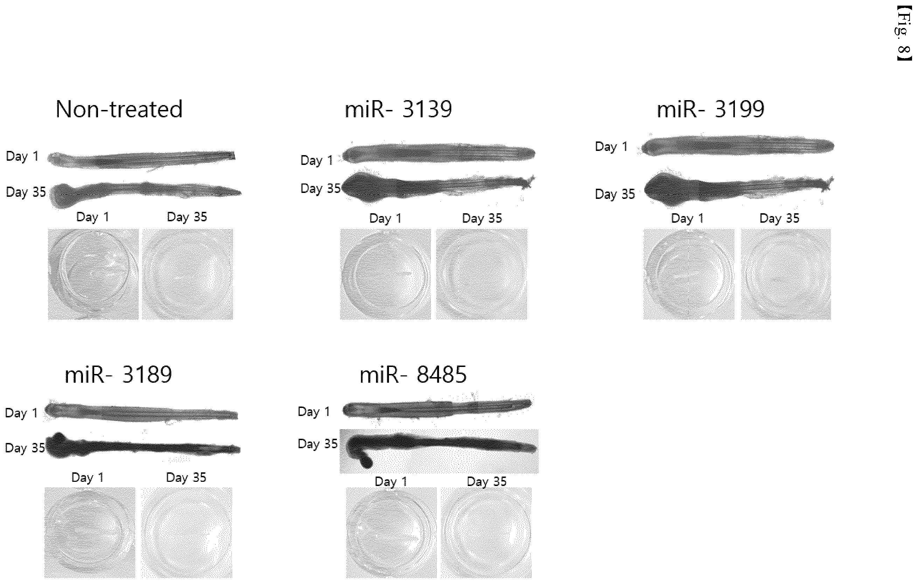

Thereby, there was no significant change during the treatment period in the non-treated gray hair, but in miR-3139, miR-3189, miR-3199 or miR-8485 treated gray hair, the outer root sheath of hair follicles widened over time, and increased proliferation or migration of hair follicle-derived primary cell including dermal papilla cell, melanocytes, and keratinocytes and also the increased length of hair follicles were observed (FIG. 7). Furthermore, it was confirmed that gray hair follicle did gradually change to brown color (FIG. 8).

### Example 4: Evaluation of melanogenic function of SAMiRNA-miR-3199 in SK-MEL-28 cells

### 4-1: Cell culture and treatment with SAMiRNA-miR-3199

SK-MEL-28 (Korean cell line bank, KR), which is a human-derived skin cancer cell line, was used to evaluate the effect of SAMiRNA-miR-3199 on melanogenesis promotion. The SK-MEL-28 cells were cultured at 37°C/5% CO₂ in MEM (Hyclone, US) supplemented with 10% FBS (Hyclone, US) and 1% penicillin-streptomycin (Hyclone, US). The SK-MEL-28 cells were dispensed at 4×10⁴ cells/well in a 12-well plate (Falcon, US) and then treated at a concentration of 5 µM the next day.

### 4-2: Analysis of amount of melanin produced by treatment with SAMiRNA-miR-3199

SK-MEL-28 cells were treated with SAMiRNA-miR-3199 by the method of Example 4-1. After culture for 72 hours, all cells were collected and color changes were observed. In order to measure the amount of melanin produced, reaction was carried out in 1 M NaOH (Bioneer, KR) for 2 hours at 37°C according to the melanin extraction protocol, after which absorbance was measured at a wavelength of 405 mm and thus the amount of melanin produced was analyzed.

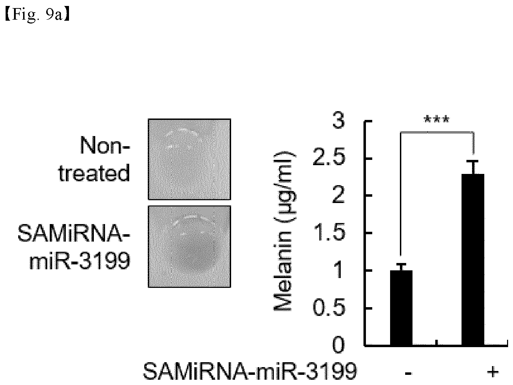

Thereby, upon treatment with SAMiRNA-miR-3199, a clear color change and an increase in the amount of melanin were observed compared to the negative control (FIG. 9a).

### 4-3: Analysis of melanogenesis signaling by treatment with SAMiRNA-miR-3199

RT-qPCR was performed for melanogenic gene analysis of MITF, TYR, TYRP1, and TYRP2 by treatment with SAMiRNA-miR-3199. The SK-MEL-28 cells were treated with SAMiRNA-miR-3199 by the method of Example 4-1. After culture for 96 hours, total RNA was extracted from the cell lysate using a Universal RNA extraction kit (Bioneer, KR), and using this RNA as a template, mRNA expression levels of the genes (Human qPCR panel kit, Bioneer, KR) and RPL13A (Human reference qPCR primer set, Bioneer, KR) were analyzed by qRT-PCR using *AccuPower* GreenStar^{™} RT-qPCR Master Mix (Bioneer, KR) according to the manufacturer's protocol. The Ct values of two genes derived after qPCR array were subjected to relative quantitative analysis through a 2(-Delta Delta C(T)) Method [Livak KJ, Schmittgen TD. 2001. Methods. Dec; 25(4):4 02-8], so that the relative amount (fold change) of each gene mRNA in the experimental group compared to the control group was calculated.

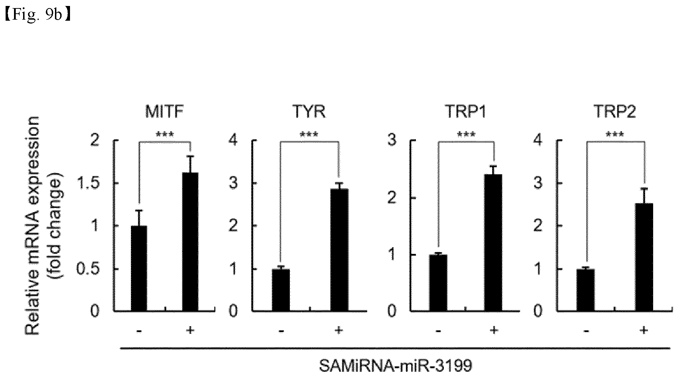

Thereby, it was confirmed that the expression of MITF, which is a key transcription factor for melanin synthesis, was significantly increased by treatment with SAMiRNA-miR-3199 and also the gene expression of TYR, TYRP1, and TYRP2, which are key enzymes for melanin synthesis, was greatly increased (FIG. 9b).

### 4-4: Analysis of melanogenic protein expression by treatment with SAMiRNA-miR-3199

Immunoblotting was performed to analyze expression of MITF and TYR proteins, which are key factors in the melanogenesis. The SK-MEL-28 cells were dispensed at 1×10⁵ cells/well in a 6-well plate (Falcon, US) and then cultured at 37°C/5% CO₂. The next day, the SK-MEL-28 cells were treated with SAMiRNA-miR-3199. After culture for 96 hours, analysis was performed according to the immunoblotting protocol. MITF (Santa Cruz Biotechnology, Inc, US), tyrosinase (Santa Cruz Biotechnology, Inc, US), and α-tubulin (Cell Signaling Technology, US) were used as primary antibodies, and HRP-linked-anti-mouse IgG (Cell Signaling Technology, US) and HRP-linked-anti-rabbit IgG (Cell Signaling Technology, US) were used as secondary antibodies.

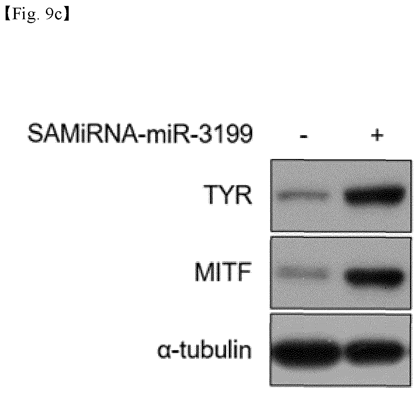

Thereby, it was confirmed that the expression of MITF and TYR proteins was significantly increased by treatment with SAMiRNA-miR-3199, like the results of gene expression analysis through RT-qPCR (FIG. 9c).

### 4-5: Analysis of intracellular expression of melanogenic protein by treatment with SAMiRNA-miR-3199

Immunocytochemistry was performed to analyze the intracellular expression of MITF and TYR proteins, which are key melanogenic factors. Before cell dispensing, a cover glass was placed in a 12-well plate (Falcon, US) and coated with poly-L-lysine (Sigma, US) for 1 hour, after which the SK-MEL-28 cells were dispensed at 2×10⁴ cells/well and cultured at 37°C/5% CO₂. The next day, the SK-MEL-28 cells were treated with SAMiRNA-miR-3199 by the method of Example 4-1. After culture for 96 hours, reaction was carried out in a 0.1% Triton-X100 solution for 10 minutes for cell permeabilization, followed by blocking in a solution containing 1% BSA (Sigma, US) for 1 hour. Here, MITF (Abeam, UK) and tyrosinase (Santa Cruz Biotechnology, Inc, US) were used as primary antibodies, and Alexa Fluor 594 anti-mouse IgG (Cell Signaling Technology, US) and Alexa Fluor 488 anti-rabbit IgG (Cell Signaling Technology, US) were used as secondary antibodies. For fluorescence analysis of the stained cells, images were analyzed using a spinning disk confocal microscope (Dragonfly high-speed confocal image platform, Andor).

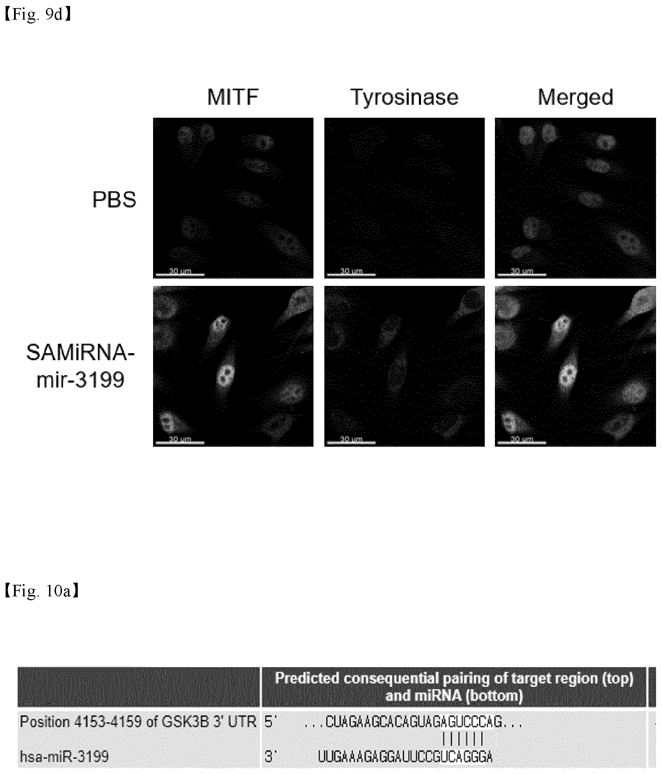

Thereby, it was confirmed that, by treatment with SAMiRNA-miR-3199, the expression of MITF, which is a transcription factor, was significantly increased in the nucleus and the expression of TYR, which is a key enzyme for melanin synthesis, was greatly increased in the cytoplasm with melanosomes (FIG. 9d).

### Example 5: Analysis of melanogenic mechanism of SAMiRNA-miR-3199

### 5-1: Identification of target gene of miR-3199

Among many genes predicted to be able to bind to the seed sequence of miR-3199 using a miRNA target gene prediction program (TargetScan, http://www/targetscan.org/), the GSK3β gene, which is known as key signaling molecule in the melanogenesis, was selected. Binding of miR-3199 to 4153-4159 of the 3'-UTR of GSK3β was predicted (FIG. 10a).

### 5-2: Analysis of inhibitory effects of SAMiRNA-miR-3199 on GSK3β mRNA

RT-qPCR was performed to analyze the effects of SAMiRNA-miR-3199 to inhibit the target gene GSK3β mRNA. The SK-MEL-28 cells were dispensed at 4×10⁴ cells/well in a 12-well plate (Falcon, US) and then cultured at 37°C/5% CO₂. The next day, the SK-MEL-28 cells were treated with SAMiRNA-miR-3199 by the method of Example 4-1. After culture for 24 hours, total RNA was extracted from the cell lysate using a Universal RNA extraction kit (Bioneer, KR), and using this RNA as a template, mRNA expression levels of GSK3β (Bioneer, KR) and RPL13A (Human reference qPCR primer set, Bioneer, KR) were analyzed by qRT-PCR using *AccuPower* GreenStar^{™} RT-qPCR Master Mix (Bioneer, KR) according to the manufacturer's protocol. The Ct values of two genes derived after qPCR array were subjected to relative quantitative analysis through a 2(-Delta Delta C(T)) Method [Livak KJ, Schmittgen TD. 2001. Methods. Dec;25(4):4 02-8], so that the relative amount (fold change) of each gene mRNA in the experimental group compared to the control group was calculated. Primer sequences for GSK3β are shown below (Table 6).

**[Table 6] Human GSK3β primer sequences**

| | |
|---|---|
| hGSK3β-forward | 5'- GCAGCATGAAAGTTAGCAGAG -3' (SEQ ID NO: 29) |
| hGSK3β-reverse | 5'- TGACTTCTTGTGGCCTGTC -3' (SEQ ID NO: 30) |

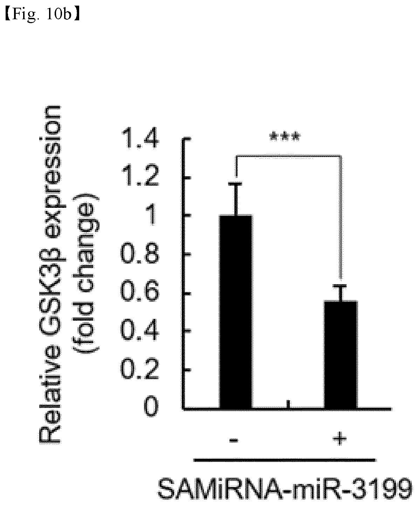

Thereby, it was confirmed that the expression of the target gene GSK3β was significantly reduced by treatment with SAMiRNA-miR-3199 (FIG. 10b).

### 5-3: Analysis of inhibitory effects of SAMiRNA-miR-3199 on GSK3β protein

In order to analyze the effects of SAMiRNA-miR-3199 to inhibit GSK3β protein, immunoblotting was performed. The SK-MEL-28 cells were dispensed at 1×10⁵ cells/well in a 6-well plate (Falcon, US) and then cultured at 37°C/5% CO₂. The SK-MEL-28 cells were treated with SAMiRNA-miR-3199 by the method of Example 4-1. After culture for 96 hours, analysis was performed according to the immunoblotting protocol. GSK3β (Cell Signaling Technology, Inc, US), β-catenin (Becton, Dickinson and Company Inc, US), and α-tubulin (Cell Signaling Technology, US) were used as primary antibodies, and HRP-linked-anti-mouse IgG (Cell Signaling Technology, US) and HRP-linked-anti-rabbit IgG (Cell Signaling Technology, US) were used as secondary antibodies.

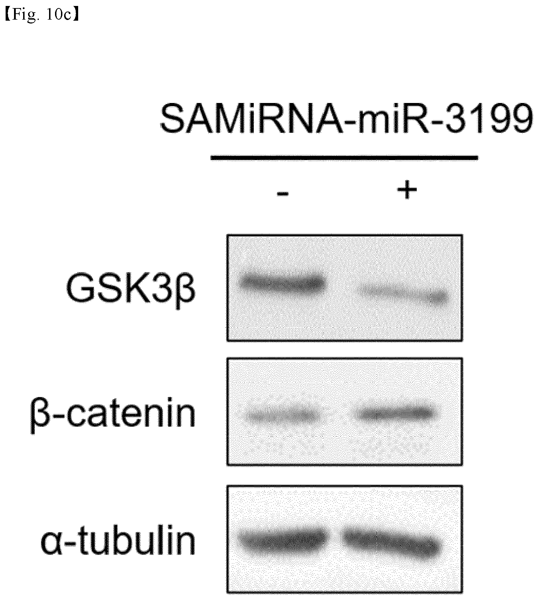

Thereby, it was confirmed that the expression of GSK3β protein was decreased by treatment with SAMiRNA-miR-3199, like the results of GSK3β gene expression analysis through RT-qPCR. GSK3β phosphorylates β-catenin to promote protein degradation, lowering protein stability, and when the expression of GSK3β is inhibited, β-catenin protein stability is increased. Therefore, it was confirmed that the expression of β-catenin protein was increased by treatment with SAMiRNA-miR-3199 (FIG. 10c).

### 5-4: Analysis of intracellular GSK3β protein expression by treatment with SAMiRNA-miR-3199

Immunocytochemistry was performed to analyze intracellular GSK3β protein expression by treatment with SAMiRNA-miR-3199. Before cell dispensing, a cover glass was placed in a 12-well plate (Falcon, US) and coated with poly-L-lysine (Sigma, US) for 1 hour, after which the SK-MEL-28 cells were dispensed at 2×10⁴ cells/well and cultured at 37°C/5% CO₂. The next day, the SK-MEL-28 cells were treated with SAMiRNA-miR-3199 by the method of Example 4-1. After culture for 48 hours, reaction was carried out in a 0.1% Triton-X100 solution for 10 minutes for cell permeabilization, followed by blocking in a solution containing 1% BSA for 1 hour. Here, GSK3β (Cell Signaling Technology, Inc, US) and β-catenin (Becton, Dickinson and Company Inc, US) were used as primary antibodies, and Alexa Fluor 594 anti-mouse IgG (Cell Signaling Technology, US) and Alexa Fluor 488 anti-rabbit IgG (Cell Signaling Technology, US) were used as secondary antibodies. Fluorescence analysis of the stained cells was performed using a spinning disk confocal microscope (Dragonfly high-speed confocal image platform, Andor).

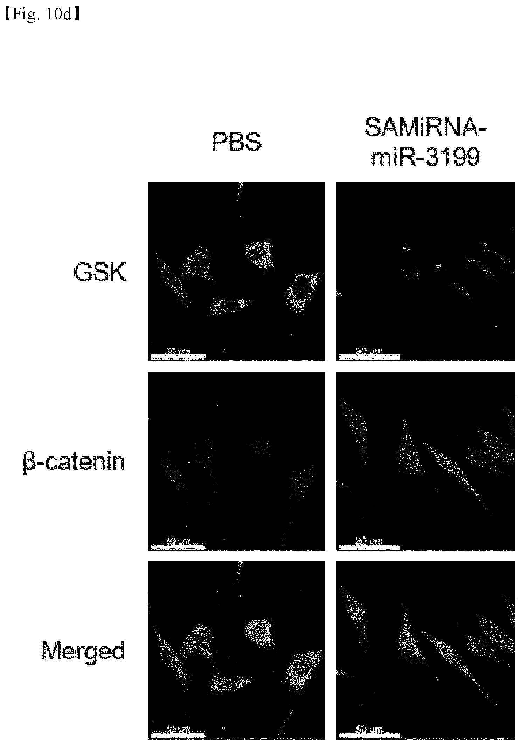

Thereby, it was confirmed that the expression of the target gene GSK3β was decreased by treatment with SAMiRNA-miR-3199 and also the expression of β-catenin protein was significantly increased in the nucleus and cytoplasm (FIG. 10d).

### 5-5: Analysis of direct binding of SAMiRNA-miR-3199 to 3'-UTR of GSK3β mRNA

In order to analyze whether miR-3199 directly binds to the 3'-UTR of GSK3β mRNA, a luciferase reporter assay was performed. The SK-MEL-28 cells were dispensed at 3×10⁴ cells/well in a 12-well plate (Falcon, US) and then cultured at 37°C/5% CO₂. The next day, the SK-MEL-28 cells were transfected with constructs into which GSK3β-wild type (WT) with a wild-type GSK3β binding site or GSK3β-mutant (MT) with a mutated GSK3β binding site were inserted using Lipofectamine RNAiMAX (Invitrogen, US). Simultaneously, the cells were treated with SAMiRNA-miR-3199. After culture for 96 hours, luciferase activity was measured and analyzed according to the manufacturer's protocol using a Dual-Luciferase^{®} Reporter Assay System (Promega^{™} Corporation, US) .

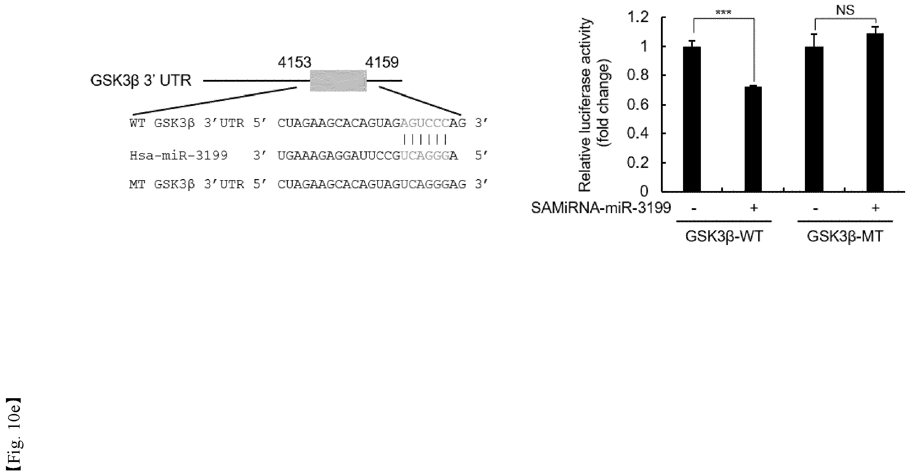

Thereby, it was confirmed that, when the cells expressing GSK3β-WT with a wild-type binding site were treated with SAMiRNA-miR-3199, luciferase activity was significantly decreased, and there was no change in luciferase activity by treatment with SAMiRNA-miR-3199 in the cells expressing GSK3β-MT with a mutant binding site (FIG. 10e). It indicates that miR-3199 inhibited GSK3β by directly binding to the 3'-UTR (4153-4159) of GSK3β mRNA.

### Example 6: Evaluation of melanogenic function of SAMiRNA-miR-3199 in human melanocytes

### 6-1: Cell culture and treatment with SAMiRNA-miR-3199

Human epidermal melanocytes (HEMs, Invitrogen, US) obtained from human wild-type skin tissue were used to evaluate the efficacy of SAMiRNA-miR-3199 on melanogenesis promotion. HEMs were cultured at 37°C/5% CO₂ in a mammalian cell culture/primary cell culture medium (Gibco, US) containing melanocyte growth supplement-2 (Gibco, US). HEMs were dispensed at 1×10⁵ cells/well in a 12-well plate (Falcon, US), and the next day, were treated with SAMiRNA-miR-3199 at a concentration of 5 µM.

### 6-2: Analysis of amount of melanin produced by treatment with SAMiRNA-miR-3199

In order to evaluate the efficacy of SAMiRNA-miR-3199, color change and the amount of melanin produced were measured in HEMs. HEMs were treated with SAMiRNA-miR-3199 at a concentration of 5 µM by the method of Example 6-1. After culture for 72 hours, all cells were collected and color changes were observed. In order to measure the amount of melanin produced, reaction was carried out in 1 M NaOH (Bioneer, KR) for 2 hours at 37°C according to the melanin extraction protocol, after which absorbance was measured at a wavelength of 405 mm and thus the amount of melanin produced was analyzed.

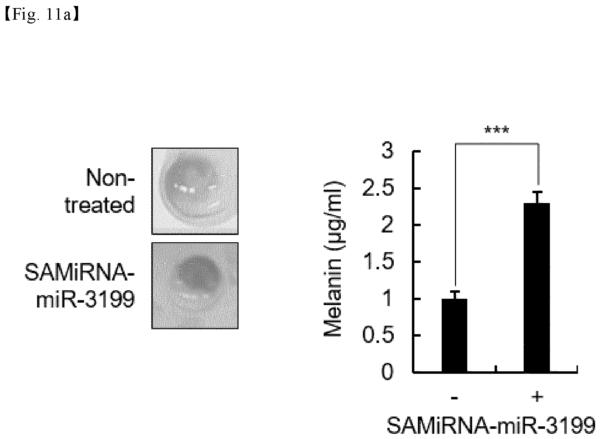

Thereby, upon treatment with SAMiRNA-miR-3199, a clear color change and an increase in the amount of melanin were observed compared to the negative control (FIG. 11a).

### 6-3: Analysis of melanogenesis signaling by treatment with SAMiRNA-miR-3199

RT-qPCR was performed for melanogenic gene expression analysis of MITF, TYR, TYRP1, TYRP2, and GSK3β after treatment with SAMiRNA-miR-3199. HEMs were treated with SAMiRNA-miR-3199 by the method of Example 6-1. After culture for 96 hours, total RNA was extracted from the cell lysate using a Universal RNA extraction kit (Bioneer, KR), and using this RNA as a template, mRNA expression levels of the genes (Human qPCR panel kit, Bioneer, KR) and RPL13A (Human reference qPCR primer set, Bioneer, KR) were analyzed by qRT-PCR using *AccuPower* GreenStar^{™} RT-qPCR Master Mix (Bioneer, KR) according to the manufacturer's protocol. The Ct values of two genes derived after qPCR array were subjected to relative quantitative analysis through a 2(-Delta Delta C(T)) Method [Livak KJ, Schmittgen TD. 2001. Methods. Dec;25(4):4 02-8], so that the relative amount (fold change) of each gene mRNA in the experimental group compared to the control group was calculated.

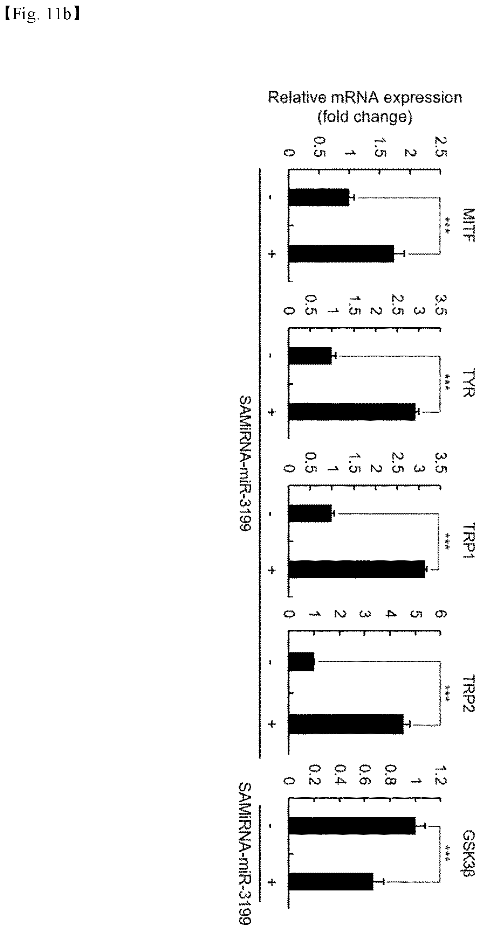

Thereby, it was confirmed that the expression of MITF, TYR, TYRP1, and TYRP2 was significantly increased by treatment with SAMiRNA-miR-3199 (FIG. 11b) and also that the expression of GSK3β, which is the target gene of miR-3199, was decreased (FIG. 11b).

### 6-4: Analysis of melanogenic protein expression by treatment with SAMiRNA-miR-3199

Immunoblotting was performed to analyze expression of MITF and TYR proteins which are key factors in the melanogenesis. HEMs were dispensed at 2×10⁵ cells/well in a 6-well plate (Falcon, US) and then cultured at 37°C/5% CO₂. HEMs were treated with SAMiRNA-miR-3199 by the method of Example 6-1. After culture for 96 hours, analysis was performed according to the immunoblotting protocol. Here, MITF (Santa Cruz Biotechnology, Inc, US), tyrosinase (Santa Cruz Biotechnology, Inc, US), and α-Tubulin (Cell Signaling Technology, US) were used as primary antibodies, and HRP-linked-anti-mouse IgG (Cell Signaling Technology, US) was used as a secondary antibody.

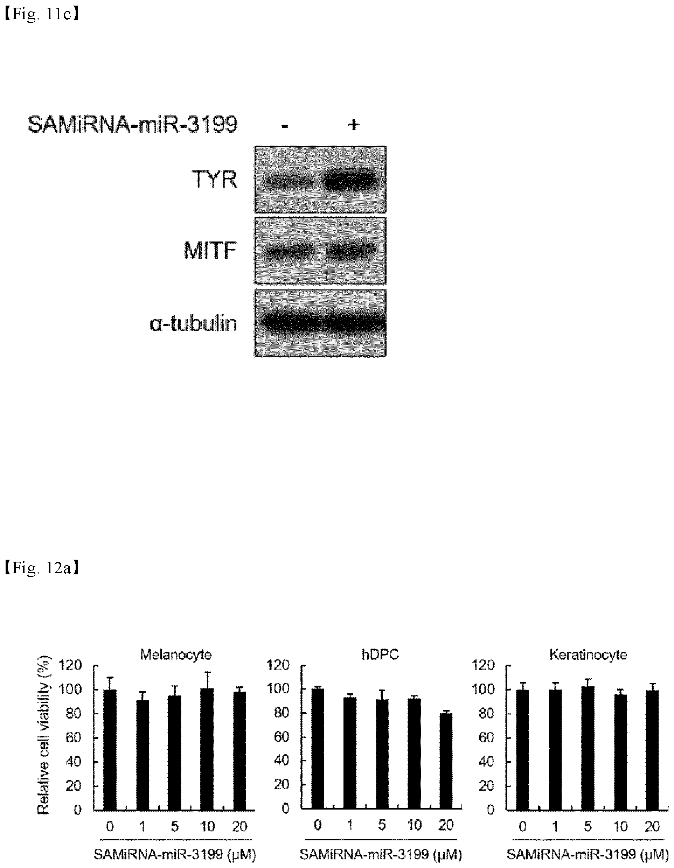

Thereby, it was confirmed that the expression of MITF and TYR proteins was increased by treatment with SAMiRNA-miR-3199, like the results of gene expression analysis through RT-qPCR (FIG. 11c).

### Example 7: Evaluation of cytotoxicity and innate immunotoxicity of SAMiRNA-miR-3199

### 7-1: Evaluation of cytotoxicity of SAMiRNA-miR-3199

In order to evaluate the cytotoxicity of SAMiRNA-miR-3199, human follicle dermal papilla cells (HFDPC, Promocell, DE), keratinocyte HaCaT (ATCC, US), and human epidermal melanocytes (Invitrogen, US) were used. HFDPC at 3×10³ cells/well, HaCaT at 4×10³ cells/well, and HEMs at 5×10³ cells/well were dispensed in a 96-well plate (Falcon, US) and then cultured at 37°C/5% CO₂. The next day, each cell was treated with SAMiRNA-miR-3199 at different concentrations (0, 1, 5, 10, and 20 µM). After culture for 72 hours, cytotoxicity was analyzed according to the manufacturer's protocol using a WST assay kit (DOGEN, KR).

Thereby, no cytotoxicity was observed in all cell lines even by SAMiRNA-miR-3199 at a high concentration of 20 µM (FIG. 12a).

### 7-2: Evaluation of innate immunotoxicity of SAMiRNA-miR-3199

In order to evaluate the innate immunotoxicity of SAMiRNA-miR-3199, human peripheral blood mononuclear cells (PBMCs) (Cellular Technology Limited, US) were used. The cells were dispensed at 5×10⁵ cells/well in a 12-well plate (Falcon, US) and then cultured at 37°C/5% CO₂. The next day, the cells were treated with SAMiRNA-miR-3199 at different concentrations (0, 5, 10, and 20 µM). After culture for 6 hours, total RNA was extracted from the cell lysate using a Universal RNA extraction kit (Bioneer, KR), and using this RNA as a template, mRNA expression levels of inflammatory cytokine genes (Human Immune qPCR panel kit, Bioneer, KR) and RPL13A (Human reference qPCR primer set, Bioneer, KR) were analyzed by qRT-PCR using *AccuPower* GreenStar^{™} RT-qPCR Master Mix (Bioneer, KR) according to the manufacturer's protocol. Primer sequences for individual genes are shown below (Table 7).

**[Table 7] Human IL-1β, IL-6, IL-12β, TNF-α, and INF-γ primer sequences**

| | | |
|---|---|---|
| hIL-1β-forward | 5'- CTGAGCTCGCCAGTGAAAT -3' | 31 |
| hIL-1β-reverse | 5'- CTGTAGTGGTGGTCGGAGA -3' | 32 |
| hIL-6-forward | 5'- AGATGCAATAACCACCCCTG -3' | 33 |
| hIL-6-reverse | 5'- TGCGCAGAATGAGATGAGTT -3' | 34 |
| hIL-12β-forward | 5'- ATTCTGGACGTTTCACCTGC -3' | 35 |
| hIL-12β-reverse | 5'- GTCCCCTCTGACTCTCTCTG -3' | 36 |
| hTNF-α-forward | 5'- CTGTAGCCCATGTTGTAGCA -3' | 37 |
| hTNF-α-reverse | 5'- GGTTATCTCTCAGCTCCACG -3' | 38 |
| hINF-γ-forward | 5'- GAATGTCCAACGCAAAGCAA -3' | 39 |
| hINF-γ-reverse | 5'- ACCTCGAAACAGCATCTGAC -3' | 40 |

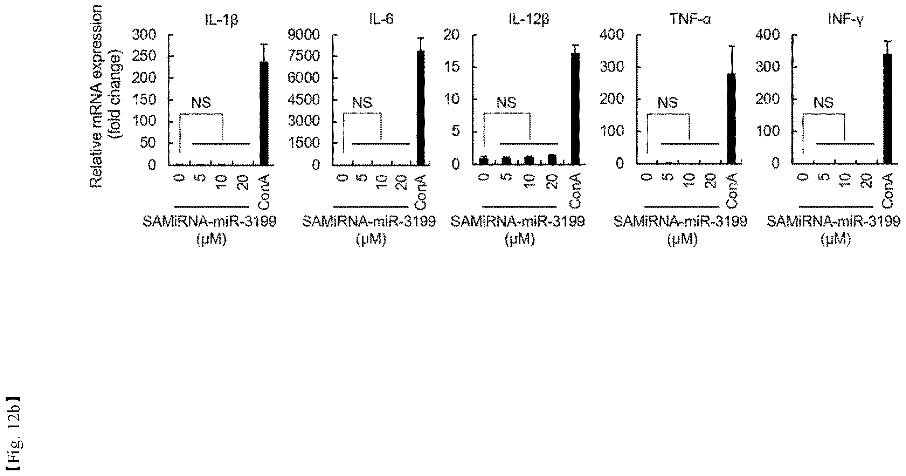

Thereby, no increase in inflammatory cytokines (IL-1β, IL-6, IL-12β, TNF-α, INF-γ, etc.) was observed even by SAMiRNA-miR-3199 at a high concentration of 20 µM, indicating no innate immunotoxicity (FIG. 12b).

### Example 8: Double-stranded oligonucleotide construct (SAMiRNA)

The double-stranded oligonucleotide construct manufactured in the present invention has the structure of Structural Formula (5) below.

C₁₈-S-S-C₆-5' S 3'-polyethylene glycol 2000 3' AS 5'-PO₄ Structural Formula (5)

In Structural Formula (5), S is the sense strand of miRNA, AS is the antisense strand of miRNA, PO₄ is a phosphate group, and polyethylene glycol is a hydrophilic material monomer, in which polyethylene glycol 2000 is linked through a phosphate group (PO₃⁻) as a linker, C₂₄ is a hydrophobic material and contains a disulfide bond, and 5' and 3' represent the terminal directions of the double-stranded oligo RNA.

For the sense strand of miRNA in Structural Formula (5), phosphodiester bonds constituting the RNA backbone structure were connected using DMT-polyethylene glycol 2000-CPG as a support and using β-cyanoethylphosphoramidite, whereby an oligo RNA-hydrophilic material construct including the sense strand in which polyethylene glycol is attached to the 3' end was synthesized, followed by attachment of hydrophobic C24 containing a disulfide bond to the 5' end, resulting in a desired sense strand of the RNA-polymer construct. For the antisense strand to be annealed with the sense strand, phosphodiester bonds constituting the RNA backbone structure were connected using β-cyanoethylphosphoramidite to form the antisense strand having a sequence complementary to the sense strand, followed by attachment of a phosphate group to the 5' end, resulting in an antisense strand.

### [Industrial Applicability]

A composition according to the present invention can activate melanocytes and promote melanogenesis, thereby preventing hair graying and slowing down the progress there of, and can convert hair that has already undergone graying into hair before graying, and thus can be efficiently utilized for pharmaceutical application and for cosmetics or hair dyeing without side effects differentiated from simple dyes that have been conventionally repeatedly used to hide hair graying. Moreover, the composition of the present invention can be used as a pharmaceutical or cosmetic composition that can induce alopecia relief and hair growth promotion without side effects by promoting proliferation of hair follicle dermal papilla cells and keratinocytes in addition to melanocyte activation.

Although specific embodiments of the present invention have been disclosed in detail above, it will be obvious to those skilled in the art that the description is merely of preferable exemplary embodiments and is not to be construed as limiting the scope of the present invention. Therefore, the substantial scope of the present invention will be defined by the appended claims and equivalents thereto.

### [Sequence List Free Text]

An electronic file is attached.

## Claims

1. A pharmaceutical composition for alleviating hair graying, promoting hair growth, and/or preventing or alleviating hair loss, comprising, as an active ingredient:
(i) miR-3139, miR-3189, miR-3199, or miR-8485;
(ii) a double-stranded oligonucleotide construct having a structure of Structural Formula (1) below:
A-X-R-Y-B Structural Formula (1)
in Structural Formula (1), A is a hydrophilic material, B is a hydrophobic material, X and Y each independently represent a simple covalent bond or a linker-mediated covalent bond, and R represents miR-3139, miR-3189, miR-3199, or miR-8485; or
(iii) nanoparticles comprising the double-stranded oligonucleotide construct.

2. The pharmaceutical composition according to claim 1, wherein the miR-3139 comprises a nucleotide sequence represented by SEQ ID NO: 9 and a nucleotide sequence represented by SEQ ID NO: 10.

3. The pharmaceutical composition according to claim 1, wherein the miR-3189 comprises a nucleotide sequence represented by SEQ ID NO: 5 and a nucleotide sequence represented by SEQ ID NO: 6.

4. The pharmaceutical composition according to claim 1, wherein the miR-3199 comprises a nucleotide sequence represented by SEQ ID NO: 15 and a nucleotide sequence represented by SEQ ID NO: 16.

5. The pharmaceutical composition according to claim 1, wherein the miR-8485 comprises a nucleotide sequence represented by SEQ ID NO: 1 and a nucleotide sequence represented by SEQ ID NO: 2.

6. The pharmaceutical composition according to claim 1, wherein the hydrophilic material is represented by (P)ₙ, (Pₘ-J)ₙ, or (J-Pₘ)ₙ, in which P is a hydrophilic material monomer, n is 1 to 200, m is 1 to 15, and J is a linker connecting m hydrophilic material monomers to each other or connecting m hydrophilic material monomers and oligonucleotides to each other.

7. The pharmaceutical composition according to claim 1, wherein the hydrophilic material has a molecular weight of 200 to 10,000.

8. The pharmaceutical composition according to claim 1, wherein the hydrophilic material is any one selected from the group consisting of polyethylene glycol (PEG), polyvinylpyrrolidone, and polyoxazoline.

9. The pharmaceutical composition according to claim 6, wherein the hydrophilic material monomer (P) has a structure of Compound (1) below: 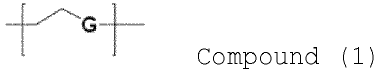 in Compound (1), G is selected from the group consisting of CH₂, O, S, and NH.

10. The pharmaceutical composition according to claim 6, wherein the linker (J) is selected from the group consisting of PO₃⁻, SO₃, and CO₂.

11. The pharmaceutical composition according to claim 1, wherein the hydrophobic material has a molecular weight of 250 to 1,000.

12. The pharmaceutical composition according to claim 11, wherein the hydrophobic material is selected from the group consisting of a steroid derivative, a glyceride derivative, glycerol ether, polypropylene glycol, a C₁₂-C₅₀ unsaturated or saturated hydrocarbon, diacylphosphatidylcholine, fatty acid, phospholipid, and lipopolyamine.

13. The pharmaceutical composition according to claim 12, wherein the steroid derivative is selected from the group consisting of cholesterol, cholestanol, cholic acid, cholesteryl formate, cholestanyl formate, and cholesteryl amine.

14. The pharmaceutical composition according to claim 12, wherein the glyceride derivative is selected from the group consisting of mono-, di-, and tri-glycerides.

15. The pharmaceutical composition according to claim 1, wherein the covalent bond represented by X and Y is a non-degradable bond or a degradable bond.

16. The pharmaceutical composition according to claim 15, wherein the non-degradable bond is an amide bond or a phosphate bond.

17. The pharmaceutical composition according to claim 15, wherein the degradable bond is a disulfide bond, an acid-degradable bond, an ester bond, an anhydride bond, a biodegradable bond, or an enzyme-degradable bond.

18. A cosmetic composition for alleviating hair graying, promoting hair growth, and/or preventing or alleviating hair loss, comprising, as an active ingredient:
(i) miR-3139, miR-3189, miR-3199, or miR-8485;
(ii) a double-stranded oligonucleotide construct having a structure of Structural Formula (1) below:
A-X-R-Y-B Structural Formula (1)
in Structural Formula (1), A is a hydrophilic material, B is a hydrophobic material, X and Y each independently represent a simple covalent bond or a linker-mediated covalent bond, and R represents miR-3139, miR-3189, miR-3199, or miR-8485; or
(iii) nanoparticles comprising the double-stranded oligonucleotide construct.

19. The cosmetic composition according to claim 18, wherein the miR-3139 comprises a nucleotide sequence represented by SEQ ID NO: 9 and a nucleotide sequence represented by SEQ ID NO: 10.

20. The cosmetic composition according to claim 18, wherein the miR-3189 comprises a nucleotide sequence represented by SEQ ID NO: 5 and a nucleotide sequence represented by SEQ ID NO: 6.

21. The cosmetic composition according to claim 18, wherein the miR-3199 comprises a nucleotide sequence represented by SEQ ID NO: 15 and a nucleotide sequence represented by SEQ ID NO: 16.

22. The cosmetic composition according to claim 18, wherein the miR-8485 comprises a nucleotide sequence represented by SEQ ID NO: 1 and a nucleotide sequence represented by SEQ ID NO: 2.

23. The cosmetic composition according to claim 18, wherein the hydrophilic material is represented by (P)ₙ, (Pₘ-J)ₙ, or (J-Pₘ)ₙ, in which P is a hydrophilic material monomer, n is 1 to 200, m is 1 to 15, and J is a linker connecting m hydrophilic material monomers to each other or connecting m hydrophilic material monomers and oligonucleotides to each other.

24. The cosmetic composition according to claim 18, wherein the hydrophilic material has a molecular weight of 200 to 10,000.

25. The cosmetic composition according to claim 18, wherein the hydrophilic material is any one selected from the group consisting of polyethylene glycol (PEG), polyvinylpyrrolidone, and polyoxazoline.

26. The cosmetic composition according to claim 23, wherein the hydrophilic material monomer (P) has a structure of Compound (1) below: 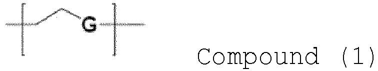 in Compound (1), G is selected from the group consisting of CH₂, O, S, and NH.

27. The cosmetic composition according to claim 23, wherein the linker (J) is selected from the group consisting of PO₃⁻, SO₃, and CO₂.

28. The cosmetic composition according to claim 18, wherein the hydrophobic material has a molecular weight of 250 to 1,000.

29. The cosmetic composition according to claim 28, wherein the hydrophobic material is selected from the group consisting of a steroid derivative, a glyceride derivative, glycerol ether, polypropylene glycol, a C₁₂-C₅₀ unsaturated or saturated hydrocarbon, diacylphosphatidylcholine, fatty acid, phospholipid, and lipopolyamine.

30. The cosmetic composition according to claim 29, wherein the steroid derivative is selected from the group consisting of cholesterol, cholestanol, cholic acid, cholesteryl formate, cholestanyl formate, and cholesteryl amine.

31. The cosmetic composition according to claim 29, wherein the glyceride derivative is selected from the group consisting of mono-, di-, and tri-glycerides.

32. The cosmetic composition according to claim 18, wherein the covalent bond represented by X and Y is a non-degradable bond or a degradable bond.

33. The cosmetic composition according to claim 32, wherein the non-degradable bond is an amide bond or a phosphate bond.

34. The cosmetic composition according to claim 32, wherein the degradable bond is a disulfide bond, an acid-degradable bond, an ester bond, an anhydride bond, a biodegradable bond, or an enzyme-degradable bond.

35. The cosmetic composition according to claim 18, wherein the cosmetic composition is selected from the group consisting of a hair tonic, hair conditioner, hair essence, hair lotion, hair nutrition lotion, hair shampoo, hair rinse, hair treatment, hair cream, hair nutrition cream, hair moisture cream, hair massage cream, hair wax, hair aerosol, hair pack, hair nutrition pack, hair soap, hair cleansing foam, hair oil, hair drying agent, hair preservative, hair dye, hair waving agent, hair bleach, hair gel, hair glaze, hair dressing, hair lacquer, hair moisturizer, hair mousse, and hair spray.
